(19) Europäisches Patentamt
European Patent Office
Office européen des brevets

(11) EP 0 736 520 B1

(12) **EUROPÄISCHE PATENTSCHRIFT**

(45) Veröffentlichungstag und Bekanntmachung des Hinweises auf die Patenterteilung:
**20.01.1999 Patentblatt 1999/03**

(51) Int. Cl.$^6$: **C07C 209/86**, C07C 209/84, C07C 211/50

(21) Anmeldenummer: **96104873.3**

(22) Anmeldetag: **27.03.1996**

(54) **Fraktionierung und Reinigung von aromatischen Polyamingemischen und deren Verwendung**
Fractionation and purification of mixtures of aromatic polyamines and their use
Fractionnement et purification de mélanges de polyamines aromatiques et leur utilisation

(84) Benannte Vertragsstaaten:
**BE DE ES FR GB IT NL PT**

(30) Priorität: **07.04.1995 DE 19513070**

(43) Veröffentlichungstag der Anmeldung:
**09.10.1996 Patentblatt 1996/41**

(73) Patentinhaber: **BAYER AG**
**51368 Leverkusen (DE)**

(72) Erfinder:
- **Knöfel, Hartmut, Dr.**
  **51519 Odenthal (DE)**
- **Brockelt, Michael**
  **51379 Leverkusen (DE)**

(56) Entgegenhaltungen:
**EP-A- 0 288 892**
**DE-A- 1 568 087**
**DE-A- 2 528 694**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 736 520 B1

## Beschreibung

Die Erfindung betrifft ein Verfahren zur Fraktionierung und Reinigung von aromatischen Polyamingemischen und deren Verwendung.

Die Herstellung von aromatischen Polyaminen und Polyamingemischen, insbesondere der Diphenylmethanreihe wird in zahlreichen Patentmeldungen und Patenten beschrieben, ebenso die Verwendung dieser Produkte. Herausragende Bedeutung kommt dabei der Verwendung dieser Produkte als Rohstoffe für die Herstellung von Isocyanaten zu, in der Regel durch Umsetzung der Polyamingemische mit Phosgen nach den allgemein üblichen und bekannten Methoden.

Die dabei resultierenden Isocyanate bzw. Isocyanatgemische fallen in vielen Fällen aber nicht in der Form und Zusammensetzung an, wie sie auf der Isocyanatstufe bevorzugt weiterverwendet werden, sondern müssen zuvor durch teilweise aufwendige Aufarbeitungs- und Trennverfahren in die verwendungsgerechte Form übergeführt werden. Geeignete Polyaminvorstufen, die weniger aufwendig in die Isocyanatverwendungsformen gebracht werden können, sind in vielen Fällen verfahrenstechnisch schwierig oder nicht zugänglich oder wirtschaftlich unattraktiv herzustellen.

Beispielhaft ist die Gewinnung des für die Herstellung hochwertiger Polyurethanwerkstoffe wichtigen 4,4'-Diisocyanato-diphenylmethans, dessen Aminvorstute in der Regel aus Anilin und Formaldehyd nur gemeinsam mit Isomeren, insbesondere dem 2,4'Isomeren, und höherfunktionellen Polyaminen gewonnen werden kann. Diese Bestandteile sind zwar die Grundlage für die ebenfalls begehrten Isocyanate, jedoch ist die Auftrennung der Rohisocyanate in die für die Weiterverwendung geeigneten Isocyanate bzw. Isocyanatgemische nicht einfach.

In der Regel werden zunächst ein Teil der Zweikernverbindungen von dem Rest abgetrennt. Anschließend wird aus der Zweikernfraktion in einem viele Trennstufen erfordernden zweiten Destillationsschritt das 4,4'-Diisocyanato-diphenylmethan von den anderen Isomeren befreit.

Das 2,4'-Isomere in angereicherter Form hat selbst in neuerer Zeit zunehmende Bedeutung als Polyurethanrohstoff erlangt, kann aber nur mit beträchtlichem destillativen Aufwand gegenüber dem 4,4'-Isomeren angereichert und von dem gegebenenfalls vorhandenen 2,2'-Isomeren befreit werden.

Isomerentrennverfahren oder Anreicherungsverfahren innerhalb der Fraktion der höherkernigen Homologen bzw. der höherfunktionellen Bestandteile der Amine wie auch der Isocyanate der Diphenylmethanreihe sind praktisch nicht bekannt.

Zunehmendes Interesse findet auch das 4,4'-Diamino-diphenylmethan als Rohstoff für das Di-(4-isocyanatocyclohexyl)-methan, die kernhydrierte Form des 4,4'-Diisocyanato-diphenylmethans, wobei die Bereitstellung geeigneter aromatischer Polyamingemische für die Hydrierstufe mit einem möglichst hohen Gehalt an 4,4'-Diamino-diphenylmethan bei gleichzeitig einem möglichst geringen Anteil an 2,4'-Diamino-diphenylmethan sehr aufwendig ist.

Es ist bekannt, daß Amine durch partielle Überführung in ihre Salze in bestimmten Fällen getrennt werden können, wobei u.a. die unterschiedlichen Basenstärken genutzt werden. Dabei handelt es sich in der Regel um Monoamine mit stark unterschiedlichen Basenstärken.

Auch für aromatische Polyamingemische, insbesondere der Diphenylmethanreihe, sind solche Disproportionierungseffekte in zweiphasigen Systemen bereits beschrieben (DE-A 2238319 und DE-A 2528697).

Die Effekte sind infolge der in einem solchen Gemisch vorhandenen zahlreichen Komponenten, deren Aminogruppen sich vom Typ her - praktisch alle sind Arylaminogruppen - kaum unterscheiden, nicht besonders groß und ausgeprägt, um für eine direkte Nutzung mit einfachen Mitteln interessant zu sein.

Aufgabe war es, ein Verfahren zur Verfügung zu stellen, das es gestattet, aromatische Polyamingemische in einfacher Weise zu fraktionieren bzw. zu reinigen, so daß Isomere in reiner Form oder in angereicherter Form anfallen.

Diese Aufgabe konnte durch das erfindungsgemäße Verfahren, welches aufgrund der erfindungsgemäßen Ausführung überraschend hohe Trennleistung bei der Fraktionierung von aromatischen Polyamingemischen, insbesondere der Diphenylmethanreihe, erzielt, und dabei in der Wirkung weit über die bekannten Effekte des Standes der Technik hinausgeht, gelöst werden.

Bei der erfindungsgemäßen Fraktionierung von aromatischen Polyamingemischen werden andere, unterschiedlich zusammengesetzte Polyamingemische gewonnen.

Bei diesen abgeleiteten Polyamingemischen kann es sich um solche handeln, die auf bekannten Synthesewegen nur sehr aufwendig zugänglich sind. Dabei kann es sich auch um Polyamingemische handeln, die für eine vereinfachte Herstellung der Isocyanate besser geeignet sind, als die bekannten und technisch gut herzustellenden Polyamingemische, indem sie z.B. auf der Isocyanatstufe schwierig durchzuführende Isomerentrennungen auf der Aminstufe vorwegnehmen. Solche Gemische können auch völlig neuartige, weil nach dem Stand der Technik nicht darstellbare Polyamingemische sein, die zu völlig neuartigen Isocyanaten führen.

Andererseits kann das erfindungsgemäße Verfahren dazu genutzt werden, aus beliebigen, d.h. auch aus wiedergewonnenen Polyamingemischen, die sich durch Verunreinigung oder durch nichtstatistische, d.h. selektive Verluste bei einzelenen Komponenten von den ursprünglich eingesetzten Polyaminen oder Isocyanaten unterscheiden, Produktfraktionen zu gewinnen, welche dem Standard oder den Ausgangspolyaminen entsprechen.

Schließlich kann das erfindungsgemäße Verfahren dazu genutzt werden, synthesebedingte und im Endprodukt unerwünschte Neben- und Zwischenprodukte, mitzufraktionieren und in einer Produktfraktion ab- und in einer anderen entsprechend anzureichern, gegebenenfalls in einer eigenen Fraktion auszuschleusen.

Gegenstand der Erfindung ist ein Verfahren zur Fraktionierung und Reinigung von aromatischen Polyamingemischen, insbesondere von Polyamingemischen der Diphenylmethanreihe, welches dadurch gekennzeichnet ist, daß man

a) das Polyaminausgangsgemisch (A) in einem zweiphasigen System bestehend aus (i) einer hydrophoben Lösungsmittelphase (B) die im wesentlichen aus hydrophobem Lösungsmittel und gegebenenfalls einem aromatischen Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, und gegebenenfalls aus Polyamin besteht, und (ii) einer wäßrigen Phase (C), bestehend im wesentlichen aus Wasser, einer starken Säure und zumindest teilweise in der Salzform vorliegendem Hilfsamin, sowie gegebenenfalls, zumindest teilweise in der Salzform vorliegenden Polyaminen, unter Zuhilfenahme einer nach dem Gegenstromprinzip arbeitenden Extraktionsstufe (4) unter Durchmischung der Phasen verteilt, indem man das Ausgangspolyamingemisch vorzugsweise über die wäßrige Phase in die Extraktionsstufe (4) einbringt und die die Extraktionsstufe (4) verlassende organische Phase (D)

b) gegebenenfalls zumindest teilweise über eine zwischengeschaltete Extraktionsstufe (3) und/oder

c) gegebenenfalls unter Abtrennung eines Teilstromes vor oder nach der gegebenenfalls durchlaufenen Extraktionsstufe (3) und Rückführung des abgetrennten Teilstromes zur Extraktionsstufe (4) über eine vorgeschaltete Extraktionsstufe (2)

d) in einer mehrstufigen Destillation (6.1), (6.2) in eine erste Fraktion (E), bestehend im wesentlichen aus hydrophobem Lösungsmittel und gegebenenfalls Hilfsamin, eine zweite Fraktion (F), bestehend im wesentlichen aus Hilfsamin und gegebenenfalls hydrophobem Lösungsmittel und einem Destillationsrückstand (G), bestehend im wesentlichen aus einer ersten Polyaminfraktion, auftrennt und

e) die die Extraktionsstufe (4) verlassende wäßrige Phase (H) in eine Extraktionsstufe (5) leitet, in welcher nach dem Prinzip der Gegenstromextraktion eine Extraktion der wäßrigen Phase mit einer aus Lösungsmittel und Hilfsamin bestehenden Lösungsmittelphase (J) erfolgt und die an Polyarylamin verarmte wäßrige Phase (K) resultiert, die

f) gegebenenfalls, zumindest teilweise, über eine zwischengeschaltete Destillationsstufe (8)

g) gegebenenfalls, zumindest teilweise, über eine zwischengeschaltete Extraktionsstufe (3) und/oder

h) gegebenenfalls zumindest teilweise zunächst über eine vorgeschaltete Extraktionsstufe (2) und anschließend über eine gegebenenfalls vorhandene Extraktionsstufe (3)

i) zur Extraktionsstufe (4) zurückgeführt und dort gegebenenfalls nach Zugabe von Wasser und/oder Hilfsamin als Mengenstrom (C) wiederverwendet wird und man

j) die in der Extraktionsstufe (5) anfallende organische Phase (L) in ein aus hydrophobem Lösungsmittel und Hilfsamin bestehendes Destillat (M) und einen, aus einer zweiten Polyaminfraktion bestehenden Destillationsrückstand (N) zerlegt, wonach das Destillat (M) mit zumindest einer Teilmenge des in der zweiten Destillationsstufe (6.2) der organischen Phase (D) gewonnenen Destillates (F) vereinigt und anschließend zur Extraktionsstufe (5) zurückgeführt und dort wiederverwendet wird.

Bevorzugt wird das Verfahren so durchgeführt, daß man

f) die die Extraktionsstufe (5) verlassende wäßrige Phase (K) vor ihrer Wiederverwendung zumindest teilweise destillativ (8) von einem Teil (X) des in ihr enthaltenen Wassers befreit, dieses gegebenenfalls zum Waschen (6.0) des der destillativen Aufarbeitung (6.1), (6.2) zugeführten Teiles der die Extraktionsstufe (4) verlassenden organischen Phase (D) und/oder der die Extraktionsstufe (3) verlassenden organischen Phase (P) und/oder zum Waschen (7.0) des der destillativen Aufarbeitung (7.1) zugeführten Teiles der die Extraktionsstufe (5) verlassenden

organischen Phase (L) zwecks Entfernens von Säurespuren verwendet, das hierbei anfallende Wasser (Y) an geeigneter Stelle in die wäßrige Phase zurückführt und die resultierende konzentrierte wäßrige Phase mit dem gegebenenfalls verbliebenen Rest von (K) vereiningt und als Mengenstrom (C) der Wiederverwendung zuführt.

Das erfindungsgemäße Verfahren wird besonders bevorzugt so durchgeführt, daß

b) die in der Extraktionsstufe (4) anfallende organische Phase (D) zumindest teilweise in einer zwischengeschalteten Extraktionsstufe (3) mit zumindest einer Teilmenge des Mengenstrom (C) im Gegenstrom extrahiert und/oder mit zumindest einer Teilmenge der in der gegebenenfalls vorhandenen vorgeschalteten Extraktionsstufe (2) anfallenden wäßrigen Phase im Gegenstrom extrahiert,
die in der zwischengeschalteten Extraktionsstufe (3) resultierende wäßrige Phase der Extraktionsstufe (4) zuführt und die in der zwischengeschalteten Extraktionsstufe (3) anfallende organische Phase der Aufarbeitungsstufe (6) zuführt.

Eine weiter verbesserte und daher bevorzugte Ausführungsform des erfindungsgemäßen Verfahrens besteht darin, daß man

c) einen Teilstrom der die Extraktionsstufe (4) verlassenden organischen Phase (D) und/oder einen Teilstrom der die gegebenenfalls vorhandene zwischengeschaltete Extraktionsstufe (3) verlassenden organischen Phase abtrennt,
und in einer vorgeschalteten Extraktionsstufe (2) mit einer Teilmenge, vorzugsweise mit der Gesamtmenge der als Mengenstrom (C) zur Verfügung stehenden wäßrigen Phase im Gegenstrom extrahiert, den in der Extraktionsstufe (2) eingesetzten organischen Mengenstrom so bemißt, daß in (2) ein möglichst weitgehender Übergang des im besagtem organischen Mengenstrom enthaltenen Polyamins in die wäßrige Phase erfolgt,
die in der vorgeschalteten Extraktionsstufe (2) resultierende wäßrige Phase gegebenenfalls nach Zugabe von Wasser aus Mengenstrom (Y) und/oder Hilfsamin der Extraktionsstufe (3) zuführt und
die in der vorgeschalteten Extraktionsstufe (2) anfallende an Polyamin verarmte organische Phase (T) der Extraktionsstufe (4) zuführt.

Als Hilfsamin wird vorzugsweise Anilin eingesetzt und als Polyamingemisch der Diphenylmethanreihe bevorzugt ein Polyamingemisch, wie es bei der säurekatalysierten Anilin-/Formaldehyd-Kondensation anfällt.

Die derart behandelten Polyamingemische, also die mit dem erfindungsgemäßen Verfahren erzeugten Fraktionen werden zur Herstellung der entsprechenden aromatischen Polyisocyanatgemische und zur Herstellung von Polyurethankunststoffen verwendet.

Außerdem können die nach dem erfindungsgemäßen Verfahren erzeugten Fraktionen zur Herstellung der entsprechenden kernhydrierten Polyamine oder als Vernetzer und als Epoxidhärter verwendet werden.

Die aus den fraktionierten Polyamingemischen hergestellten entsprechenden Polyisocyanate werden bevorzugt zur Herstellung von PU-Schaumstoffen eingesetzt.

Ausgangsgemische sind beispielsweise technische Arylamingemische, wie sie bei der Herstellung aus den Ausgangsverbindungen oder wie sie bei der Wiedergewinnung anfallen.

Beispielsweise von Ausgangsarylamingemischen, für deren Fraktionierung und Reinigung das erfindungsgemäße Verfahren besonders geeignet ist, sind

1. Polyamingemische der Diphenylmethanreihe, wie sie bei der Kondensation und säurekatalysierten Umlagerung von Anilin mit Formaldehyd entstehen,

2. Polyamingemische der Diphenylmethanreihe, wie sie bei der säurekatalysierten Kondensation von substituierten Anilinen mit Formaldehyd anfallen,

3. Polyamingemische der Diphenylmethanreihe, wie sie bei der Mischkondensation von substituierten Anilinen untereinander und/oder mit Anilin mit Formaldehyd anfallen,

4. Polyamingemische der Diphenylmethanreihe, wie sie bei der Kondensation, auch der Mischkondensation von substituierten Anilinen und/oder Anilin mit Aldehyden und/oder Ketonen anfallen,

5. Polyamingemische der Diphenylmethanreihe, wie sie bei der Nitrierung und anschließenden Reduktion von Di- und/oder Polyarylmethanen und/oder substituierten Di- und/oder Polyarylmethanen entstehen; unter Polyarylmethanen werden hier insbesondere die Benzylhomologen des Diphenylmethans verstanden,

6. Polyamingemische der Diphenylmethanreihe, wie sie bei der Kondensation von Monoarylmonoaminen (z.B. Anilin, substituierte Aniline) und/oder Monoaryldiaminen (Phenylendiamine, substituierte Phenylendiamine) mit Aldehyden, Ketonen, insbesondere Formaldehyd, und säurekatalysierter Umlagerung entstehen und

7. Polyamingemische der Triphenylmethanreihe, wie sie z.B. bei der Nitrierung und anschließenden Reduktion von Triphenylmethan, insbesondere alkylsubstituierten Triphenylmethanen und seinen höherkernigen, insbesondere Benzylhomologen entstehen.

Bei den zum Einsatz gelangenden hydrophoben Lösungsmitteln handelt es sich um inerte Lösungsmittel des Siedepunktbereiches von 30 - 280°C, vorzugsweise von 80 - 200°C, wie beispielsweise Chlorbenzol, Dichlorbenzol, Benzol, Toluol, Ethylbenzol, Cumol, Xylol, Dichlorethan, Chloroform und Tetrachlorkohlenstoff. Vorzugsweise werden Xylole, d.h. technische Xylolgemische, insbesondere o-Xylol, Toluol, Ethylbenzol, Cumol und Chlorbenzol eingesetzt.

Bevorzugt werden solche Lösungsmittel verwendet, die ein gutes Lösungsvermögen für die eingesetzten Polyamingemische aufweisen.

Bei den eingesetzten Säuren handelt es sich um wasserlösliche Protonsäuren mit einem unter 2,5, vorzugsweise unter 1,5 liegenden pKA-Wert. Beispiele hierfür sind Salzsäure, Bromwasserstoffsäure, Schwefelsäure, Trifluoressigsäure, Methansulfonsäure oder Phosphorsäure.

Bevorzugt eingesetzt werden Salzsäure und Methansulfonsäure. Die genannten Säuren können auch im Gemisch mit sauren oder neutralen Salzen derartiger Säuren, wie z.B. den entsprechenden Ammoniumsalzen oder auch den entsprechenden Alkalisalzen, eingesetzt werden. Die genannten Säuren werden nicht in freier Form eingesetzt, sondern liegen in dem erfindungsgemäßen Kreislaufsystem in Form der entsprechenden Ammoniumsalze der in den wäßrigen Kreislaufsystem befindlichen Basen vor. Das sind in der Regel Polyamingemische von der Art der Ausgangsgemische und/oder die verwendeten Hilfsamine.

Als Hilfsamin finden in der Regel Monoarylamine, wie z.B. Anilin und/oder am Kern und/oder am Stickstoff alkylsubstituierte Anilinderivate, Verwendung. Bevorzugt werden primäre Aniline eingesetzt, besonders bevorzugt ist Anilin.

Das erfindungsgemäße Verfahren kann sowohl diskontinuierlich als auch kontinuierlich durchgeführt werden. Bevorzugte Ausführungsform ist die kontinuierliche Arbeitsweise. Dabei wird das Verfahren in allen Stufen unter dem Eigendruck des Systems und vorzugsweise in einer Inertgasatmosphäre (Stickstoff) durchgeführt.

Das erfindungsgemäße Verfahren kann zur Steigerung des Anreicherungs-bzw. korrespondierenden Abreicherungseffektes mit jeder der anfallenden Produktfraktionen wiederholt werden.

Das erfindungsgemäße Verfahren kann sowohl mit zwei (Abb.1) als auch mit drei (Abb.2 und Abb.3) oder mit vier (Abb.4) Extraktionsstufen durchgeführt werden.

Die in Abb. 1 - 4 dargestellten Fließdiagramme dienen der weiteren Erläuterung des erfindungsgemäßen Verfahrens. In diesen Abbildungen bedeuten:

(1) einen Tank für das Ausgangsarylamingemisch
(2) eine vorgeschaltete Extraktionsstufe
(3) eine erste Extraktionsstufe
(4) eine (zwischengeschaltete) zweite Extraktionsstufe
(5) eine (letzte) dritte Extraktionsstufe
(6) eine Aufarbeitungsstufe bestehend aus

  (6.0) einer Waschstufe
  (6.1) einer ersten Destillationsstufe einer Mehrstufendestillation
  (6.2) einer letzten Destillationsstufe einer Mehrstufendestillation

(7) eine weitere Aufarbeitungsstufe bestehend aus

  (7.0) einer Waschstufe
  (7.1) eine Destillationsstufe

(8) einen Wasserverdampfer
(9) einen Tank für Verfahrensprodukt
(10) einen Tank für ein weiteres Verfahrensprodukt

Die Bezugszeichen A-U, X und Y bezeichnen die Mengenströme auf die nachstehend und in den Beispielen Bezug genommen wird.

Die vorgeschaltete Extraktionsstufe (2) wird vorzugsweise als mehrstufig wirkender Extraktor ausgeführt.

Die gegebenenfalls zwischengeschaltete Extraktionsstufe (3) besteht im einfachsten Falle ebenfalls aus einer Mischer-Scheidereinheit, vorzugsweise kommen jedoch auch hier mehrstufig wirkende Extraktionseinheiten zum Einsatz.

Bei der Extraktionsstufe (4) handelt es sich im einfachsten Fall um eine einstufig wirkende Mischer-Scheidereinheit, vorzugsweise kommen jedoch mehrstufig wirkende Extraktionseinheiten zum Einsatz.

Die letzte Extraktionsstufe (5) wird im allgemeinen als mehrstufig wirkender Extraktor ausgeführt.

Die Aufarbeitungsstufen (6) und (7) dienen der Abtrennung der Polyaminfraktion, welche als Destillationsrückstände anfallen und als Verfahrensprodukte (G) und (N) in den Tanks (9) und (10) isoliert werden, und der Wiedergewinnung des eingesetzten hydrophoben Lösungsmittels und des verwendeten Hilfsamins als Destillate.

Es hat sich als zweckmäßig erwiesen, die den Destillationsstufen zugeführten organischen Phasen (D) bzw. (D') und (L) bzw. (L') vor ihrer destillativen Behandlung in vorgelagerten Waschstufen (6.0) und (7.0) durch Extraktion mit Wasser von anhaftenden Säurespuren zu befreien.

Die eigentliche Aufarbeitungsstufe (6) besteht im allgemeinen aus einer wenigstens zweistufigen Mehrstufendestillation deren erste Stufe (6.1) ein gegenüber dem Zulaufprodukt (D) bzw. (D') und/oder (P) bzw. (P') von Polyamin befreites und an Hilfsamin verarmtes hydrophobes Lösungsmittel als Destillat (E) liefert, und deren letzte Stufe (6.2) ein gegenüber (D) bzw. (D') und/oder (P) bzw. (P') verarmtes Hilfsamin als Destillat (F) liefert.

Die vollständige destillative Auftrennung von hydrophoben Lösungsmittel und Hilfsamin ist bei der Durchführung des erfindungsgemäßen Verfahrens nicht erforderlich.

Zusätzlich fällt in der letzten Destillationsstufe (6.2) die in Mengenstrom (D) bzw. (D') und/oder (P) bzw. (P') enthaltene erste Polyaminfraktion des Ausgangsgemisches (A) als Destillationssumpf (G) an.

Die Aufarbeitungsstufe (7) besteht im einfachsten Fall aus einer Destillationskolonne (7.1), die so ausgelegt ist, daß eine weitgehende Abtrennung von hydrophobem Lösungsmittel und Hilfsamin gemeinsam als Destillat (M) von der im Zulauf zu (6) enthaltenen Polyaminfraktion (N) destillativ erfolgt.

Vorzugsweise wird jedoch auch die Aufarbeitungsstufe (7) des erfindungsgemäßen Verfahrens wegen der verbesserten Energienutzung als mehrstufige Destillation ausgelegt.

Bei der Destillationsstufe (8) handelt es sich um eine Vorrichtung, mit welcher der wäßrigen Phase des Systems oder einem Teilstrom der wäßrigen Phase destillativ Wasser entzogen werden kann.

Ein solcher Schritt ist für die Durchführung des erfindungsgemäßen Verfahrens grundsätzlich nicht erforderlich, wegen der sich ergebenden Vorteile sind die Ausführungsformen unter Einschluß einer Wasserdestillationsstufe (8) jedoch bevorzugt.

Bei der wäßrigen, die Säure enthaltenden Phase liegt praktisch ein geschlossenes Kreislaufsystem vor, so daß die Stufe (8) grundsätzlich an einer beliebigen Stelle dieses Kreislaufsystems eingeschoben werden kann. Die Position von Stufe (8) in Anschluß an die Extraktionsstufe (5) und vor Eintritt in die Extraktionsstufe (4) ist die vorteilhafteste und daher die bevorzugte Ausführungsform.

Die entnommene Wassermenge (X) wird gegebenenfalls nach ihrer Aufteilung in Teilströme und deren unterschiedlicher Verwendung, in Form des Mengenstromes (Y) insgesamt oder in Teilströmen dem System an geeigneter Stelle wieder zugeführt, so daß ein erweitertes und gegebenenfalls verzweigtes in sich geschlossenes wäßriges Kreislaufsystem entsteht.

Dieses schließt auch die Waschstufen (6.0) und/oder (7.0) mit ein. Letztere sind ein oder mehrstufig wirkende nach dem Gegenstromprinzip arbeitende Extraktionsstufen. In Waschstufe (6.0) wird die organische Phase (D) bzw. (D') und/oder (P) bzw. (P') mit einem Teilstrom von (X) von anhaftenden Säurespuren befreit, in der Waschstufe (7.0) wird die organische Phase (L) bzw. (L'), unter Verwendung eines anderen Wasserteilstromes von (X), von anhaftenden Säurespuren befreit.

Das mit hydrophoben Lösungsmittel und Hilfsamin contaminierte Destillat (X) ist für die Waschstufen (6.0) und (7.0) bestens geeignet. Die resultierenden Waschwässer weisen in der Regel eine sehr viel geringere Säurekonzentration auf als der eigentliche Säurekreislauf, so daß diese problemlos in Form des Mengenstromes (Y) oder seiner Teilströme recyclisiert werden können, gegebenenfalls kann eine Destillatteilmenge von (X) von (8) an den Waschstufen vorbei nach (Y) geführt und zur Aussteuerung eines unterschiedlichen Wassergehaltes der wäßrigen Phase in den einzelnen Extraktionsstufen verwendet werden.

Die praktisch quantitative Kreislaufführung der verwendeten Säure ermöglicht den Einsatz kostspieliger Säuren wie z.B. Methansulfonsäure, die wiederum wegen ihrer geringeren Korrosionsneigung die Verwendung kostengünstiger Werkstoffe in den Apparaten des erfindungsgemäßen Verfahrens gestattet.

Es hat sich als zweckmäßig erwiesen, den Säuregehalt der wäßrigen Phase, unabhängig von dem sich in der wäßrigen Phase eines zweiphasigen Systems einstellenden unterschiedlichen Amingehalt, über eine sogenannte "Molarität" zu definieren.

Die "Molarität " wird festgelegt als theoretische Konzentration von zu 100% protoniertem Amin (d.h. gleiche Anzahl von Säure- und Aminäquivalenten) in einem rechnerisch um den Anteil an nicht protoniertem Amin verminderten Volumen an wäßriger Phase gemäß der Formel

$$\text{"Molarität"} = \frac{\text{Mol 100\% protoniertes Amin}}{\text{Vol. wäßrige Phase - Vol. unprotoniertes Amin}}$$

Die so definierte Molarität kann Werte bis 6 annehmen und wird je nach der jeweiligen Ausführungsform zugrunde liegenden - hier produktbezogenen - Trennaufgabe in diesem Bereich gezielt variiert.

Auch innerhalb einer Ausführungsform des erfindungsgemäßen Verfahrens ist es gegebenenfalls vorteilhaft, die einzelnen von der wäßrigen Phase durchlaufenen Verfahrensstufen, insbesondere die Extraktionsstufen (2) bis (5) mit unterschiedlicher Molarität in der wäßrigen Phase zu betreiben, indem der wäßrigen Phase zwischen den einzelnen Stufen Wasser entzogen oder zugeführt wird.

Nach oben wird dieser Arbeitsbereich technisch begrenzt einerseits durch zunehmende Kristallisationsneigung der Aminsalze mit zunehmender Konzentration, insbesondere bei hohen Protonierungsgraden, und andererseits durch die zunehmende gegenseitige Löslichkeit der Phasen ineinander, insbesondere bei niedrigen Protonierungsgraden.

Der Protonierungsgrad gibt das Verhältnis von Säureäquivalenten zu Aminäquivalenten wieder.

Nach unten wird dieser Bereich wirtschaftlich begrenzt durch den abnehmenden Säuregehalt und damit die quantitative Abnahme der Trennleistung, d. h. bei hervorragender qualitativer Trennleistung und technisch problemlos, ist mit sinkender Molarität ein zunehmend größeres Volumen an wäßriger Phase erforderlich für die Trennung einer gegebenen Aminmenge.

Gemäß einer Variante des erfindungsgemäßen Verfahrens erfolgt die Einspeisung des Ausgangspolyamingemisches (A) aus dem Vorratsbehälter (1) durch Vermischen mit dem Teilstrom (C) und Einführung des Gemisches in die Extraktionsstufe (4).

Im allgemeinen besteht der Mengenstrom (C) aus Wasser, einer starken Protonsäure, Hilfsamin und gegebenenfalls Polyamin.

Die Säure liegt in Form ihrer in Wasser gelösten Salze mit Hilfsamin und gegebenenfalls mit Polyamin vor. Die Aminogruppen von Hilfsamin und gegebenenfalls Polyamin liegen in (C) stets im stöchiometrischen Überschuß vor, bezogen auf die Säure.

Der Protonierungsgrad beträgt in (C) im allgemeinen 10 - 90 %, für das vorzugsweise als Hilfsamin verwendete Anilin liegt er vorzugsweise bei 25 - 70 %.

Die für die jeweilige Ausführungsform des erfindungsgemäßen Verfahrens wohldefinierte und in engen Grenzen gemessene und geregelte Molarität des Mengenstromes (C) wird je nach der der jeweiligen Ausführungsform zugrunde liegenden - hier produktbezogenen - Trennaufgabe in einem weiten Bereich gezielt variiert. Im allgemeinen hat die der Extraktionsstufe (4) des erfindungsgemäßen Verfahrens zugeführte wäßrige Phase einen Molarität zwischsen 0,5 und 6.

Im Extraktor (4) wird dem aus (A) und (C) erzeugten Gemisch der Mengenstrom (B) entgegengeführt.

Die organische Phase (B) besteht im allgemeinen neben hydrophobem Lösungsmittel aus Hilfsamin und/oder Polyamin, letzteres vorzugsweise mit der Zusammensetzung des zweiten Verfahrensteilproduktes (N).

Bei Verwendung einer organischen Phase (B) ohne Polyamin resultiert in der die Extrationsstufe (4) verlassenden wäßrigen Phase (H) eine Polyaminfraktion, in welcher die relative Anreicherung der in dieser Phase bevorzugt enthaltenen Komponenten gezielt erhöht und maximiert werden kann, auf Kosten der Polyaminkonzentration in der wäßrigen Phase.

Polyamin als Bestandteil der organischen Phase (B) bewirkt, daß die die Verfahrensstufe (4) verlassende Phase (H) eine höhere und damit für die Durchführung des erfindungsgemäßen Verfahrens energetisch vorteilhaftere Polyaminkonzentration aufweisen, als bei Verwendung einer organischen Phase (B) ohne Polyamin.

Durch die bevorzugte Verwendung eines Polyamins mit der Zusammensetzung des zweiten Teilproduktes (N) als Bestandteil der organischen Phase (B) kann auch auf einem höheren und damit vorteilhaften Konzentrationsniveau infolge Gleichgewichtseinstellung mit Selbstverstärkung des Trenneffektes die relative Anreicherung der in der die Trennstufe (4) verlassenden wäßrigen Phase (H) bevorzugt enthaltenen Polyaminkomponenten und damit der zweiten Polyaminfraktion (N) variiert und maximiert werden.

Im einfachsten Fall wird Mengenstrom (B) gebildet aus dem von Polyarylamin (G) befreiten und an Hilfsaminen verarmten hydrophoben Lösungsmittel, welches als Destillatstrom (E) in der Destillationsstufe (6.1) gewonnen wird und welchem gegebenenfalls eine Teilmenge des von Polyamin (G) befreiten und an hydrophoben Lösungsmittel verarmten Destillatstromes (F) der Destillationsstufe (6.2) zugesetzt wird.

Bei einer bevorzugten Ausführungsform wird zumindest eine Teilmenge des Destillatstromes (E) und gegebenenfalls zumindest eine entsprechende zusätzliche Teilmenge des Destillatstromes (F) dem als Extraktionsmittel in der letzten Extraktionsstufe (5) eingesetzten Mengenstrom (J) zugeschlagen und nach Durchlaufen der Stufe (5) der resultierenden organischen Phase (L) als Teilmenge entnommen und dem Mengenstrom (B) zugesetzt.

Gegebenenfalls wird in einer besonders günstigen aber nicht allgemein anwendbaren Ausführungsform die Gesamtmenge der Mengenströme (E) und (F) dem Mengenstrom (J) zugeschlagen und zunächst als Extraktionsmittel in Extraktionsstufe (5) verwendet, so daß der Mengenstrom (B) ausschließlich aus einer Teilmenge des die Extraktionsstufe (5) verlassenden Mengenstromes (L) gebildet wird.

In der Regel beträgt der Gehalt der organischen Phase (B) an aromatischem Amin je nach Trennaufgabe 15 - 60 %.

In der vorzugsweise mehrstufig betriebenen Extraktionsstufe (4) werden die organische Phase (B) und das Gemisch aus Ausgangspolyamingemisch und wäßriger Phase (C) unter inniger Durchmischung einander entgegengeführt.

Bei diesem Vorgang findet in der Regel ein teilweiser Übergang von Polyamin in die organische Phase statt, gegebenenfalls im Austausch von Hilfsamin in entgegengesetzter Richtung.

Das zusammen mit der wäßrigen Phase (C) in den Extraktor (4) eingebrachte Ausgangspolyamin (A) verteilt sich auf die den Extraktor verlassende wäßrige Phase (H) und die den Extraktor (4) verlassende organische Phase (D) (quantitative Fraktionierung).

Die mengenmäßige Aufteilung der einzelnen Komponenten des Ausgangspolyamingemisches auf die resultierende wäßrige Phase (H) und die resultierende organische Phase (D) erfolgt unter den Bedingungen des erfindungsgemäßen Verfahrens mit einer überraschend hohen Selektivität, so daß die resultierenden Produktfraktionen eine andere, unter Umständen stark von der des Ausgangspolyamingemisches abweichende Zusammensetzung aufweisen (qualitative Fraktionierung).

Beispielsweise ausgehend von den bevorzugt eingesetzten Anilinformaldehydkondensationsprodukten wurde gefunden, daß von einer in zwei oder mehreren isomeren Formen im Ausgangsgemisch enthaltenen Polyaminkomponente in der Regel die ortho-isomere(n) Form(en) in der die Trennstufe (4) verlassenden organischen Phase (D) relativ angereichert ist (sind); beispielsweise 2,4'-Diamino-diphenylmethan relativ zu 4,4'-Diamino-diphenylmethan. Umgekehrt ist die resultierende wäßrige Phase (H) relativ verarmt an dem 2,4'-Isomeren, während das 4,4'-Isomere relativ angereichert ist.

Sind mehrere "ortho-Isomere" im Ausgangspolyamin vorhanden, z. B. 2,2'-und 2,4'-Diamino-diphenylmethan, dann ist das "ortho-reichere" 2,2'-Isomere in der organischen Phase (D) gegenüber dem "ortho-ärmeren" 2,4'-Isomeren stärker angereichert, welchletzteres seinerseits gegenüber dem "noch ortho-ärmeren" 4,4'-Isomeren relativ angereichert ist.

Der zuerst bei den Anilinformaldehydkondensationsprodukten der Diamino-diphenylmethanreihe gefundene An- und Abreicherungseffekt wurde rein empirisch-deskriptiv mit dem Kriterium der ortho- und para-Substitution verbunden. Die davon abgeleitete Charakterisierung der Verfahrensprodukte als "orthoreich" und "orthoarm" ist dabei relativ und wurde durch den Begriff "ortho-Substitutionsgrad" ausgedrückt.

Als "ortho-Substitutionsgrad" wird dabei das Verhältnis der orthoständigen Aminogruppen-Methylengruppenrelationen zur Gesamtzahl aller Aminogruppenrelationen definiert. Mit diesem Begriff lassen sich praktisch alle Isomerentrennungen bei den Polyaminen erfassen, die aus Arylaminen, auch substituierten, mit Carbonylverbindungen in wäßrig saurem Medium hergestellt werden.

Überraschenderweise wurde nun der gleiche An- und Abreicherungseffekt - geordnet nach ortho-Substitutionsgrad - auch für die gut charakterisierten und analytisch erfaßbaren isomeren Dreikernverbindungen aus der Anilin-Formaldehydkondensation gefunden.

Analoges gilt für die Trennung von Kondensationsprodukten aus Formaldehyd mit Anilin und Diaminoarylverbindungen wie Phenylendiamin oder alkylsubstiuierten Phenylendiaminen.

Die bisher erwähnten Polyamingemische weisen, bedingt durch ihre Herstellung, Aminogruppen auf, die praktisch nur orthoständig und/oder paraständig zu Methylengruppen sind.

Dabei werden innerhalb einer Gruppe isomerer Verbindungen in der Regel diejenigen mit dem höheren ortho-Substitutionsgrad gegenüber den Isomeren mit einem geringeren ortho-Substitutionsgrad bei der Fraktionierung in der organischen Phase (D) angereichert.

Polyamingemische insbesondere der Diphenylmethanreihe einschließlich der jeweiligen höherkernigen Homologen, die nach anderen Verfahren hergestellt werden, beispielsweise durch Nitrierung von Diphenylmethan oder Methyldiphenylmethanen und anschließende Reduktion weisen außer ortho- und para-ständigen Aminogruppen herstellungsbedingt auch andere Aminogruppen-Methylengruppenrelationen auf. Für diese Polyamingemische ist das erfindungsgemäße Verfahren genauso wirksam.

Beispielsweise läßt sich aus einem Gemisch von 2- und 4-Methyldiphenylmethan durch Nitrierung und anschließende Reduktion ein Polyamingemisch herstellen, welches in der Hauptsache ein Isomerengemisch darstellt aus

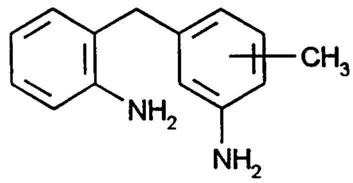

und

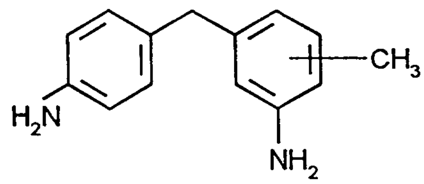

Bei der Fraktionierung solcher Gemische mit Hilfe des erfindungsgemäßen Verfahrens werden die 3,2'-Isomeren in der organischen Phase (D) gegenüber den 3,4'-Isomeren angereichert.

Das Kriterium "orthoreich" und "orthoarm" oder der ortho-Substitutionsgrad erfaßt in diesen Polyamingemischen nicht mehr alle Isomeren und ist daher sinngemäß anzuwenden, indem anstelle der Begriffe "orthoständig" und "paraständig" eine Einteilung der Isomeren vorgenommen wird in solche mit kleinerem (= ortho-) und solche mit größerem (= para-) räumlichen Abstand der - in der Regel an unterschiedlichen Sechsringen befindlichen - Aminogruppen zur Methylenbrücke bzw. der Aminogruppen zueinander.

Eine weitere Klasse aromatischer Polyamingemische, die sich mit Hilfe des erfindungsgemäßen Verfahrens sehr wirksam fraktionieren lassen, stellen die Polyamine des Triphenylmethans und seiner höherkernigen Homologen, vorzugsweise Benzylhomologen dar, wie sie z. B. durch Nitrierung und anschließende Reduktion der entsprechenden Kohlenwasserstoffgemische hergestellt werden.

Bei der Fraktionierung technischer Polyamingemische der zuletzt genannten Substanzklassen

I. Mischkondensationsprodukte von Mono- und Diaminoarylverbindungen mit Formaldehyd bzw. allgemeinen Carbonylverbindungen,

II. Polyamingemische aus Verfahren durch Nitrierung und anschließende Reduktion von Diphenylmethan und vorzugsweise substituierten insbesondere alkylsubstituierten Diphenylmethanen und den jeweiligen Homologen und

III. Polyamingemische aus Verfahren durch Nitrierung und anschließende Reduktion von Triphenylmethan und vorzugsweise substituierten insbesondere alkylsubstituierten Triphenylmethanen und den jeweiligen höherkernigen Benzylhomologen
wurde zusätzlich zur reinen Isomerentrennung eine weitere überraschende Selektivität gefunden.

Polyamingemische der genannten Substanzklasse I bis III enthalten oder können enthalten, Komponenten, bei denen wenigstens ein Arylkern pro Molekül mehr als eine, in der Regel zwei Aminogruppen trägt. Diese Komponenten können die bevorzugten Bestandteile des Polyamingemisches sein, ohne daß sie verfahrensbedingt mengenmäßig die Hauptprodukte sein müssen.

Zur besseren Charakterisierung solcher Komponenten wird der Begriff "Aminosubstitutionsgrad" verwendet, mit dem in erster Linie die Anzahl der Aminogruppen einer Komponente im Verhältnis zur Anzahl der Arylkerne gekennzeichnet wird.

Für Anilin und seine Kondensationsprodukte mit Formaldehyd ist dieser Ausdruck stets 1,0 ,für Phenylendiamin und seine Kondensationsprodukte stets 2,0. Für reine Mischkondensate ergeben sich für die Diphenylmethanisomeren der Wert 1,5 und für die höherkernigen Homologen Werte zwischen > 1,0 und < 2,0. Bei statistischer Verwendung des Begriffs Aminosubstitutionsgrad zur Charakterisierung von technischen Polyamingemischen ergeben sich ebenfalls Werte zwischen 1,0 und 2,0.

Bei der Fraktionierung von Polyamingemischen mit einem Aminosubstitutionsgrad > 1,0 wurde nun gefunden, daß die Komponenten mit einem höheren Aminosubstitutionsgrad in der im eigentlichen Trennschritt resultierenden wäßrigen Phase relativ angereichert werden, und zwar um so stärker je größer der Aminosubstitutionsgrad ist.

Somit eröffnet das erfindungsgemäße Verfahren auch für diese Substanzklasse die Möglichkeit, die Herstellungsform der Rohstoffe (Aminstufe) und die Verwendungsform der Endprodukte (Isocyanatstufe) zu entkoppeln, so daß eine getrennte Optimierung beider Stufen erleichtert wird bis hin zur Gewinnung völlig neuer Isocyanatgemische.

Ergänzt werden diese "Leistungen" durch ein weiteres Selektivitätskriterium, welches bei der Fraktionierung technischer Polyamingemische, insbesondere solcher mit höherkernigen Homologen, gefunden wurde und die "Kernigkeit" der Polyamingemische betrifft.

Mit dem Begriff "Kernigkeit" wird primär die Anzahl der Aryleinheiten einer Komponente eines aromatischen Polyamingemisches ausgedrückt. Im weiteren Sinne wird der Begriff der Kernigkeit dafür verwendet, um für ein aus zahlreichen Komponenten, mit einer individuellen exakten Kernigkeit, bestehendes Polyamingemisch statistisch eine Kernigkeit des Gesamtgemisches auszudrücken.

Besonders überraschenderweise wurde nun bei der Fraktionierung von Polyamingemischen mit höherkernigen Anteilen, insbesondere bei der Fraktionierung technischer Gemische von Anilin-Formaldehydkondensaten, gefunden, daß sich die höherkernigen Komponenten in der die Fraktionierungsstufe verlassenden organischen Phase gezielt sowohl relativ anreichern als auch relativ abreichern lassen, in Abhängigkeit von der Molarität der wäßrigen Phase in der Extraktionsstufe (4).

Eine hohe Molarität der wäßrigen Phase in (4) innerhalb des angegebenen Molaritätsbereichs führt zu einer relativen Abreicherung höherkerniger Komponenten in der organischen Phase (D) und dementsprechend zu einer relativen Anreicherung in der wäßrigen Phase (H).

Eine niedrige Molariät der wäßrigen Phase in (4) innerhalb des angegebenen Molaritätsbereiches führt zu einer relativen Anreicherung höherkerniger Komponenten in der organischen Phase (D).

Der überraschende Befund kann dahingehend erweitert und präzisiert werden, das die relative An- und Abreicherung auch innerhalb der höherkernigen Homologen untereinander stattfindet. Werden beispielsweise in einem technischen Gemisch des Diamino-diphenylmethans in der einen Fraktion die Dreikernkomponenten gegenüber den Zweikernkomponenten relativ an- oder abgereichert, wird auch eine relative An- oder Abreicherung von Vierkernkomponenten gegenüber Dreikernkomponenten, d. h. eine noch stärkere relative An- oder Abreicherung, gefunden, desgleichen von Fünfkernkomponenten gegenüber Vierkernkomponenten usw.

Daraus und aus der gleichzeitig und stets im Sinne einer relativen Verstärkung des "ortho-Substitutionsgrades" in der organischen Phase (D) ablaufenden Isomerentrennung und aus der Möglichkeit, mit einzelnen Produktfraktionen die erfindungsgemäße Trennung, gegebenenfalls mit geänderten Verfahrensparametern, zu wiederholen, ergeben sich zahlreiche Möglichkeiten, ausgehend von bekannten und gut zugänglichen Polyamingemischen über das erfindungsgemäße Verfahren zu weniger gut zugänglichen oder völlig neuen, weil nach dem Stand der Technik bislang unzugänglichen, Polyaminen und damit Polyisocyanaten zu gelangen. Das gilt besonders für Produkte, der Diamino- und Diisocyanatodiphenylmethanreihe und ganz besonders für Polyamin- und Polyisocyanatgemische mit einem extrem hohen Anteil an höherkernigen Komponenten.

Die An- bzw. Abreicherung wird in der Regel effektiver mit steigendem Protonierungsgrad in der wäßrigen Phase der Trennstufe.

Darüberhinaus erweist sich das erfindungsgemäße Verfahren als von allgemeiner Wirksamkeit auch auf andere strukturähnliche Polyamine.

So können beispielsweise in den bereits erwähnten Polyamingemischen, die durch Nitrierung von Di- und Polyarylmethanen und anschließender Reduktion gewonnen werden, auch Monoaminopolyarylmethanverbindungen oder Komponenten enthalten sein, bei denen eine oder mehrere Methylengruppen durch Nebenreaktionen in Keto- und/oder Hydroxymethylengruppen und damit in unerwünschte Nebenprodukte umgewandelt worden sind.

Bei der Kondensation von Arylaminen mit Carbonylverbindungen können zahlreiche unvollständig umgelagerte Zwischenverbindungen und Nebenprodukte auftreten.

Die meisten dieser Verbindungen unterliegen in der Regel bei der Fraktionierung der sie enthaltenden Polyamingemische einer Anreicherung in einer der resultierenden Fraktionen, so daß der Effekt zur Abtrennung und Fraktionierung genutzt werden kann.

Gegebenenfalls können derartige Produkte auf diesem Wege angereichert werden oder als gezielt hergestellte Polyamingemische, wie z. B. Polyaminobenzophenone oder Aminobenzylarylamingemische ihrerseits fraktioniert werden.

Die die Extraktionsstufe (4) verlassende, organische Phase (D) enthält unter anderem noch geringe Mengen an Säure, im allgemeinen und in Abhängigkeit von den Verfahrensparametern in der Extraktionsstufe (4) zwischen 0,01 und 0,5 Gew.-%, die vorteilhaft vor der destillativen Aufarbeitung des Mengenstroms (D) entfernt werden.

Im einfachsten Falle geschieht dies durch Neutralisation mit überschüssigen, verdünnten wäßrigen Basen, beispielsweise verdünnter Natronlauge. Bevorzugt wird jedoch das Herauswaschen der Säure bzw. ihrer Aminsalze aus der organischen Phase mit Wasser, so daß gegebenenfalls nur noch verbleibende Spuren durch Kontakt mit verdünnter Natronlauge oder mit Hilfe eines Ionentauschers entfernt werden.

Das eingesetzte Waschwasser wird unter Einschaltung eines Wasserverdampfers dem wäßrigen Säurekreislauf entzogen und diesem nach Durchlaufen der Waschstufe(n) mitsamt der Säure an verfahrensmäßig geeigneter Stelle wieder zugesetzt.

Die organische Phase (D) wird, gegebenenfalls nach Durchlaufen der Säurewaschstufe (6.0), in die wenigstens zweistufige Destillationsstufe (6.1), (6.2) überführt.

In der ersten Destillationsstufe (6.1) wird ein Destillat (E) abgetrennt, welches die Hauptmenge, vorzugsweise nahezu die Gesamtmenge des in (D) enthaltenen, hydrophoben Lösungsmittels neben einem Teil des in (D) enthaltenden Hilfsamins umfaßt. Im allgemeinen enthält das Destillat (E) < 50 % Hilfsamin, vorzugsweise 15 - 30 %.

In der letzten Destillationsstufe (6.2) wird das verbleibende Hilfsamin, gegebenenfalls neben der Restmenge des hydrophoben Lösungsmittels, als Destillat (F) von dem als Destillationssumpf anfallenden und im Verfahrensprodukttank (9) gesammelten ersten Teilprodukt (G) abgetrennt. Im allgemeinen enthält (F) < 50 % hydrophobes Lösungsmittel, vorzugsweise 15 - 30 %.

Das entsprechende zweite Verfahrensteilprodukt befindet sich in der die Extraktionsstufe (4) verlassenden wäßrigen Phase (H).

In einer mehrstufig wirkenden, vorzugsweise bei 80 - 110°C betriebenen Extraktionsstufe (5) wird aus der wäßrigen Phase (H), gegebenenfalls nach Zugabe von Wasser zur Absenkung der Molarität und gegebenenfalls nach Zugabe von Hilfsamin zur Absenkung des Protonierungsgrades, das zweite Teilprodukt im Austausch gegen Hilfsamin extrahiert und dabei in die organische Phase (L) überführt.

Die Molarität der in (5) eingesetzten wäßrigen Phase beträgt vorzugsweise < 2,5.

Der Protonierungsgrad der in (5) eingesetzten wäßrigen Phase ist vorzugsweise < 60 %.

Als Extraktionsmittel (J) dient ein Gemisch aus hydrophobem Lösungsmittel und Hilfsamin, welches im wesentlichen aus der Destillatfraktion (M) der Destillationsstufe (7.1) und zumindest einer Teilmenge der Destillatfraktion (F) der Destillationsstufe (6.2) zusammengesetzt wird und gegebenenfalls durch zumindest einer Teilmenge des Destillatstromes (E) ergänzt wird.

Das Gewichtsverhältnis von Hilfsamin zu Lösungsmittel liegt in (J) im allgemeinen zwischen 0,5 : 1 und 3 : 1, vorzugsweise zwischen 1 : 1 und 2 : 1.

Das Gewichtsverhältnis von Extraktionsmittel (J) zu wäßriger Phase liegt im allgemeinen zwischen 0,3 : 1 und 3 : 1, vorzugsweise zwischen von 0,7 : 1 und 2 : 1.

Die in (5) resultierende, organische Phase (L) oder deren Teilmenge wird, gegebenenfalls nach Durchlaufen der Waschstufe (7.0) und/oder gegebenenfalls nach Entfernen von Säurespuren mit verdünnter Natronlauge, der Destillationsstufe (7.1) zugeführt.

In der Destillationsstufe (7.1) erfolgt die destillative Abtrennung des Destillationsrückstandes (N), der als zweites Teilprodukt in dem Verfahrensprodukttank (10) gesammelt wird.

Die Destillationsstufe (7.1) kann beispielsweise aus einem einstufigen Verdampfer bestehen, der neben dem Destillationsrückstand (N) ein Destillat (M) liefert.

Das Destillat (M) enthält neben Hilfsamin das gesamte hydrophobe Lösungsmittel aus (L) oder deren Teilmenge und wird nach Zusatz zumindest einer Teilmenge von (F) als Extraktionsmittel (J) verwendet. Für den allgemeinen Fall, daß (F) neben Hilfsamin auch hydrophobes Lösungsmittel enthält, erfolgt gegebenenfalls ein entsprechender Bilanzausgleich an hydrophobem Lösungsmittel hin zu (E), d.h. zu dem organischen Kreislauf zur Gewinnung des ersten Teilproduktes.

Die in (5) resultierende wäßrige Phase (K) wird zur Extraktionsstufe (4) zurückgeführt, vorzugsweise zumindest teilweise über eine Destillationsstufe (8), in welcher der wäßrigen Phase die Wassermenge (X) entzogen wird.

Der Weg über (8) ist dann zwingend erforderlich, wenn die Extraktionsstufe (4) bei einer höheren Molarität der wäßrigen Phase betrieben wird als die Extrationsstufe (5). In diesem Falle erfolgt die zumindest anteilmäßige Rückspeisung von Wasser (Y) in den wäßrigen Säurekreislauf nach Verlassen der Stufe (4) und vor Eintritt in die Stufe (5).

Grundsätzlich kann die in Verfahrensstufe (4) resultierende wäßrige Phase (H) direkt der Extraktionsstufe (5) zugeführt werden. Da aber der obere, für bestimmte Trennaufgaben durchaus bevorzugte Molaritätsbereich der Extraktionsstufe (4) oberhalb des bevorzugten Molaritätsbereiches der Extraktionsstufe (5) liegt, ist es eine bevorzugte Ausführungsform des erfindungsgemäßen Verfahrens, die Molaritäten der in den verschiedenen Extraktionsstufen dadurch zu entkoppeln, daß dem in sich geschlossenen System der wäßrigen Phase gegebenenfalls an geeigneter Stelle destillativ Wasser entzogen und an anderer geeigneter Stelle wieder zugesetzt wird.

Mit Hilfe einer Wasserdestillationsstufe (8) wird der wäßrigen, die Säure enthaltende Phase, oder einem Teilstrom der wäßrigen Phase, vorzugsweise nach Verlassen der Extraktionsstufe (5) und vor der Wiederverwendung am Prozeßbeginn Wasser entzogen, welches vor Eintritt der wäßrigen Phase in die Extraktionsstufe (5) insgesamt (Y) oder in Teilmengen an einer oder mehreren Stellen wieder zugesetzt wird.

Vorteilhafter und als Ausführungsform bevorzugt ist eine zweite Variante des erfindungsgemäßen Verfahrens, bei der sich zusätzlich auch in der ersten Polyaminfraktion (G) die relevante Anreicherung der in dieser Fraktion bevorzugt enthaltenen Komponenten beträchtlich steigern und gezielt variieren läßt, indem die in der Extraktionsstufe (4) anfallende organische Phase (D) zumindest teilweise in einer aus der Sicht der Phase (D) zwischengeschalteten Extraktionsstufe (3) mit einer wäßrigen Phase extrahiert wird, die im vorliegenden Fall der Variante 2 im wesentlichen aus zumindest einer Teilmenge des Mengenstromes (C) besteht.

Aus formalen Gründen wird die der Extraktionsstufe (3) zugeführte organische Phase als Mengenstrom (O) bezeichnet, auch wenn sie gegebenenfalls wie im vorliegenden Fall beispielhaft ausgeführt zumindest in der Zusammensetzung, vorzugsweise aber auch in der Menge mit Mengenstrom (D) übereinstimmt.

Bereits bei der einstufigen Durchführung der Extraktionsstufe (3), beispielsweise als Mischer-Scheidereinheit, kommt es in der resultierenden organischen Phase (P) in Abhängigkeit von Art und insbesondere Menge der eingesetzten wäßrigen Phase zu einer deutlichen weiteren relativen Anreicherung der bereits in (D) gegenüber Ausgangspolyamin (A) angereicherten Komponenten, verbunden mit einer Abnahme des Polyamingehaltes in der organischen Phase (P). Vorzugsweise wird jedoch wegen der besseren Effektivität auch die zwischengeschaltete Extraktionsstufe (3) als mehrstufig wirkender und im Gegenstrom betriebener Extraktor ausgeführt.

Die in der Extraktionsstufe (3) anfallende wäßrige Phase (Q) enthält die entsprechende andere Fraktion des mit Mengenstrom (O) eingebrachten Polyamins, in der die in (P) angereicherten Komponenten entsprechend abgereichert sind. Ausmaß der relativen Abreicherung, d. h. die Zusammensetzung des in (Q) enthaltenen Polyamins wird unter den jeweiligen Verfahrensbedingungen der mehrstufig wirkenden Extraktionsstufe (3) kontrolliert durch das qualitative und quantitative Verteilungsgleichgewicht zwischen der zugeführten organischen Phase (O) und der abgeführten wäßrigen Phase (Q).

Die Molarität der wäßrigen Phase in der Extraktionsstufe (3) liegt je nach Trennaufgabe höher oder auf gleicher Höhe oder niedriger bezogen auf die Molarität in der aus Sicht der wäßrigen Phase nachgeschalteten Extraktionsstufe (4) und wird durch Zugabe oder Entzug von Wasser an geeigneter Stelle geregelt.

Die in der Verfahrensstufe (3) resultierende wäßrige Phase (Q) wird gegebenenfalls nach Zugabe von Wasser zusammen mit den gegebenenfalls vorhandenen Rest von (C) der Extraktionsstufe (4) zugeführt.

Die in Stufe (3) resultierende organische Phase (P) wird zusammen mit dem gegebenenfalls vorhandenen Rest von (D) der Aufarbeitungsstufe (6) zugeführt zur Gewinnung der Polyaminfraktion (G).

Mit der zweiten Variante des erfindungsgemäßen Verfahrens läßt sich die relative Anreicherung in beiden resultierenden Polyaminfraktionen gezielt variieren und maximieren. Neben dieser in qualitativer Hinsicht großen Vielseitigkeit und Leistungsfähigkeit bietet die zweite Verfahrensvariante zumindest für die zweite Polyaminfraktion (N) auch eine energetisch günstige Ausführungsform.

Der mit Gewinnung der ersten Polyaminfraktion (G) verbundene Energieaufwand steigt dagegen relativ zu so stärker, je geringer der mengenmäßgie Anteil von (G), bezogen auf eingesetztes Polyamingemisch (A) ist, weil der verbleibende Gehalt an Polyamin (G) in der destillativ aufzuarbeitenden organischen Phase (D) bzw. (P) gegebenenfalls immer kleiner wird.

Der Effekt kommt besonders dann zum Tragen, wenn die mit (G) abgetrennten Komponenten im Ausgangsgemisch (A) nur in geringer Konzentration enthalten sind und/oder relativ hoch in der Fraktion (G) angereichert werden, z. B. bei der erfindungsgemäßen Auftrennung von Polyamingemischen der Diphenylmethanreihe.

Eine in dieser Hinsicht verbesserte Ausführungsform stellt die dritte Variante des erfindungsgemäßen Verfahrens dar. Ausgehend von der ersten Variante, wird diese dahingehend erweitert, daß die die Verfahrensstufe (4) verlassende organische Phase (D) geteilt wird in einem Teilstrom, der weiterhin mit dem Ziel der Gewinnung der Polyaminfraktion (G) der Aufarbeitungsstufe (6) zugeführt wird und einem zweiten Teilstrom, der einer vorgeschalteten Extraktionsstufe (2) zugeführt wird.

Aus formalen Gründen wird die der vorgeschalteten Extraktionsstufe (2) zugeführte organische Phase als Mengenstrom (R) bezeichnet, auch wenn sie gegebenenfalls wie im vorliegenden Fall beispielhaft ausgeführt in der Zusammensetzung mit Mengenstrom (D) übereinstimmt.

Bei der Extraktionsstufe (2) handelt es sich in der Regel um einen mehrstufig wirkenden und im Gegenstrom betriebenen Extraktor, in welchem die zugeführte organische Phase (R) mit zumindest einer Teilmenge, vorzugsweise mit der Gesamtmenge der zu Wiederverwendung zur Verfügung stehenden wäßrigen Phase (C) extrahiert wird.

Der dem Extraktor (2) zugeführte Mengenstrom (R) wird dabei so bemessen, daß bei der Umsetzung mit Mengenstrom (C) ein möglichst weitgehender, vorzugsweise praktisch quantitativer Übergang der in der organischen Phase (R) enthaltenen Polyamine in die den Extraktor (2) verlassende wäßrige Phase (S) erfolgt.

Eine höhere Molarität in der in Stufe (2) eingesetzten wäßrigen Phase, resultierend aus Verfahrensstufe (8) des erfindungsgemäßen Verfahrens, begünstigt und erleichtert den Übergang von Polyaminen aus der organischen Phase (R) in die wäßrige Phase.

Der Restgehalt an Polyamin in der die Verfahrensstufe (2) resultierenden organischen Phase (T) liegt im allgemeinen bei < 5 Gew.-%, vorzugsweise bei < 1 Gew.-%.

Im übrigen richtet sich der in (T) zulässige Höchstgehalt an Amin und insbesondere der Gehalt an Polyamin nach den aus der jeweiligen Trennaufgabe resultierenden qualitativen Anforderungen an die Verfahrensprodukte, im Falle der Variante 3 insbesondere an das Verfahrensteilprodukt (N). Die Einhaltung des für die Qualität von (N) relevanten

Gehaltes an Polyamin wird im Rahmen der technischen Gegebenheiten unter Ausschöpfung der zur Verfügung stehenden wäßrigen Phase über die Bemessung des Teilmengenstromes (R) kontrollliert.

Dabei kommt es dem Verfahren und insbesondere der Extraktionsstufe (2) zustatten, daß die für den Einsatz in Stufe (2) zur Verfügung stehende wäßrige Phase (Mengenstrom C) um so größer ist, je größer der Anteil der zweiten Polyaminfraktion (N) und je kleiner demzufolge der Anteil der ersten Polyaminfraktion (G) ist. Eine kleine Polyaminfraktion (G) bedeutet in der Regel eine niedrige Polyaminkonzentration in der organischen Phase (D) und hohen Energieaufwand bei der Aufarbeitung einer solchen Phase. Durch die erfindungsgemäße Variante 3 kann gegenüber Variante 1 insbesondere der Energieaufwand bei der Isolierung der ersten Polyaminfraktion (G) reduziert werden.

Der Beitrag der Verfahrensstufe (2) im Rahmen der Variante 3 zur Verbesserung des erfindungsgemäßen Verfahrens besteht darin, daß für die destillative Aufarbeitung (6) zur Gewinnung der ersten Polyaminfraktion (G) anstelle des Gesamtstromes mit einer relativ niedrigen und damit energetisch ungünstigen Konzentration an Polyamin nur ein Teilstrom mit entsprechend erhöhter und damit energetisch günstigerer Konzentration anfällt (quantitative Anreicherung), während aus dem anderen Teilstrom eine an geeigneter Stelle als Extraktionsmittel verwendbare organische Phase (T) ohne Destillation gewonnen wird.

Die von Polyamin weitgehend befreite, die Verfahrensstufe (2) verlassende organische Phase (T) wird der Extraktionsstufe (4) zugeführt.

In der vorzugsweise mehrstufig wirkenden Extraktionsstufe (4) wird die organische Phase (T) als Extraktionsmittel zugesetzt, in der Regel durch Vermischen mit Mengenstrom (B) und Zugabe zu der aus der Sicht der organischen Phase (B) ersten Stufe des Extraktors.

In Abhängigkeit von einem gegebenenfalls vorhandenen Restgehalt an Polyamin in (T) und mit Rücksicht auf die Qualität der zweiten Polyaminfraktion (N) erfolgt die Zugabe der organischen Phase (T) gegebenenfalls zu einer aus Sicht der organischen Phase (B) späteren, gegebenenfalls zur letzten Stufe der mehrstufig arbeitenden Extraktionsstufe (4).

Die die Verfahrensstufe (2) verlassende wäßrige Phase (S) enthält neben der in Form ihrer Ammoniumsalze vorliegenden Säure noch Hilfsamin und Polyamin, letzteres mit einer Zusammensetzung, die weitgehend dem Polyamin in der zugeführten organischen Phase (R) entspricht.

Im Falle der Variante 3 des erfindungsgemäßen Verfahrens wird der Mengenstrom (S) direkt der Verfahrensstufe (4) zugeführt, gegebenenfalls nach Zugabe von Wasser aus Mengenstrom (Y) und/oder weiterer wäßriger Phase aus Mengenstrom (C).

Da die in der wäßrigen Phase (S) enthaltende Polyaminfraktion in der Regel eine höhere relative (qualitative) Anreicherung im Sinne der ersten Polyaminfraktion (G) bezogen auf das Ausgangspolyamin (A) aufweist, resultiert für die der Extraktionsstufe (4) zugeführte wäßrige Phase nach Zugabe von Ausgangspolyamin (A) ein gegenüber diesem in Abhängigkeit vom Mengenverhältnis "angereichertes" Mischpolyamin. Infolge des Verteilungsgleichgewichtes zwischen zugeführter wäßriger und resultierender organischer Phase (D) ergibt daraus für Variante 3 auch ein begrenzter qualitativer Anreicherungseffekt für die erste Polyaminfraktion (G).

In einer weiteren Variante 4 des erfindungsgemäßen Verfahrens werden die technischen Maßnahmen der vorausgegangenen Varianten zusammengefaßt und miteinander kombiniert.

Im einfachsten Fall werden die Extraktionsstufen (2) und (3) hinzugefügt und jede für sich mit einem Teilstrom von (C) und einem Teilstrom von (D), der in diesem Fall in drei Teilströme aufgeteilt wird, in der beschriebenen Weise durchgeführt.

Vorteilhafter ist es, als organische Phase (R) in Extraktionsstufe (2) einen Teilstrom des eine qualitativ hochangereicherte, quantitativ weniger konzentrierte Polyaminfraktion enthaltenden Mengenstromes (P) einzusetzen.

Bevorzugt wird die Variante 4 so durchgeführt, daß als organische Phase (R) in der Extraktionsstufe (2) ein Teilstrom von (D) und/oder vorzugsweise ein Teilstrom von (P) eingesetzt wird und die in Stufe (2) resultierende wäßrige Phase (S) zumindest teilweise, vorzugsweise insgesamt der Extraktionsstufe (3) zugeführt und gegebenenfalls unter Zugabe von weiterer wäßriger Phase aus Mengenstrom (C) und gegebenenfalls von Hilfsamin in (3) eingesetzt wird. Dabei wird ihr unter inniger Durchmischung in mehreren Stufen die organische Phase (O) mit ihrem Gehalt an im gleichen Sinne angereicherten Polyamin entgegengeführt, gegebenenfalls wird die organische Phase (O) verstärkt durch Zugabe eines Teilstromes der in der Extraktionsstufe (2) resultierenden organischen Phase (T) zu (O).

Durch diese Maßnahmen kommt es in der in (3) resultierenden organischen Phase (P) zu einer weiteren Steigerung des qualitativen Anreicherungseffektes. In quantitativer Hinsicht kann dieses Ergebnis durch Bemessung und Aufteilung der Mengenströme bei einem relativ hohen und damit energetisch günstigen Polyamingehaltes in den in (3) resultierenden Phasen, insbesondere in der organischen Phase (P) erreicht werden.

Die Rückkopplung des Anreicherungseffektes in (P) über dem Mengenstrom (R) als Teilstrom von (P) und über die wäßrige Phase (S) wirkt dabei selbstverstärkend.

Durch die erfindungsgemäße Ausführung und Verschaltung der Extraktionsstufe (2) bis (4) in Variante (4) mit Verfahrenskriterien wie Disproportionierung anstelle von fraktionierter Extraktion in Stufe (3), mit Selbstverstärkung durch Verschaltung mit Extraktionsstufe (2) und Wiedergewinnung von Extraktionsmittel in Stufe (2) ohne Destillation für den

Einsatz in der Verfahrensstufe (4) und gegebenenfalls in (3) resultiert ein Maximum an qualitativer Trennleistung, die in Kombination mit der Variation der Molarität der wäßrigen Phasen in den Stufen (2) bis (4) zu einer großen Anwendungsbreite des erfindungsgemäßen Verfahrens führt.

**Beispiel 1**

Ausgangspolyamingemisch (A) (4,900 kg/h) wird mit dem im wesentlichen aus Polyamingemisch, Anilin, Chlorwasserstoff und Wasser bestehenden Mengenstrom (C) (19,307 kg/h) vermischt.

Mengenstrom (C) wird gebildet aus den Mengenströmen (U) und (Q).

Die resultierende wässrige Phase (Mengenströme A+C) hat im Mittel folgende Zusammensetzung

Mengenstrom (A)+(C) ( 24,207 kg/h )
28,6 % Polyarylamin
18,9 % Anilin
5,8 % Chlorwasserstoff
46,7 % Wasser.

und wird in einem mehrstufig wirkenden Extraktor (4) bei 90°C dem Mengenstrom (B) entgegengeführt, der die folgende Zusammensetzung hat:

Mengenstrom (B) ( 9,429 kg/h )
29,7 % Anilin
69,2 % Xylol
ca. 1,1 % Wasser.

Die den Extraktor (4) verlassende organische Phase (D) wird insgesamt den Extraktor (3) zugeführt und ist in diesem Falle nach Menge und Zusammensetzung identisch mit Mengenstrom (O). Mengenstrom (D) bzw. (O) hat folgende durchschnittliche Zusammensetzung:

Mengenstrom (D) bzw. (O) ( 12,106 kg/h )
22,8 % Polyarylamin
22,3 % Anilin
53,9 % Xylol
< 0,1 % Chlorwasserstoff
ca. 1,0 % Wasser.

Die den Extraktor (4) verlassende wässrige Phase (H) hat folgende durchschnittliche Zusammensetzung:

Mengenstrom (H) ( 21,530 kg/h )
19,4 % Polyarylamin
21,7 % Anilin
6,5 % Chlorwasserstoff
52,4 % Wasser.

Mengenstrom (H) wird mit Mengenstrom (Y) ( ca. 2,9 kg/h ) vereinigt und in dem mehrstufig wirkenden Extraktor (5) bei 90°C dem organischen Mengenstrom (J) entgegengeführt.

Mengenstrom (H)+(Y) ( 24,441 kg/h )

Mengenstrom (J) ( 21,958 kg/h )
54,9 % Anilin
44,1 % Xylol
ca. 1,0 % Wasser

Mengenstrom (J) wird gebildet aus dem Destillatstrom (M) der Destillationsstufe (7.1) und einem Teilmengenstrom von (F).

Die den Extraktor (5) verlassende organische Phase (L) fällt an mit der folgenden durchschnittlichen Zusammensetzung:

Mengenstrom (L) ( 25,000 kg/h )
16,0 % Polyarylamin
44,1 % Anilin
38,8 % Xylol
< 0 , 1 %
Chlorwasserstoff
ca. 1,0 % Wasser.

In einer als mehrstufiger Extraktor ausgeführten Waschstufe (7.0) wird der Mengenstrom (L) im Gegenstrom mit Wasser (ca. 2 kg/h) gewaschen.

Zur Sicherheit wird der gewaschene Mengenstrom (L) mit verdünnter Natronlauge gewaschen. Die wässrige Phase wird als Abwasser entsorgt.

Im anschließenden Destillationsschritt (7.1) werden Wasser, Xylol und Anilin von der als Destillationssumpf anfallenden Polyaminfraktion ( Mengenstrom N ) abgetrennt.

Das in der Destillationsstufe (7.1) anfallende Destillat - gegebenenfalls nach mechanischer Abtrennung des beim Abkühlen des Destillates abgeschiedenen Wassers - wird als Mengenstrom (M) mit einem Teilstrom des im wesentlichen aus Anilin bestehenden Mengenstromes (F) aus der Destillationsstufe (6.2) vereinigt unter Bildung des als Extraktionsmittel in der Extraktionsstufe (5) eingesetzten Mengenstromes (J).

Die Polyaminfraktion (N) wird als orthoarmes Teilprodukt mit ca. 4,0 kg/h in Tank (10) gesammelt.

Die den Extraktor (5) verlassende wässrige Phase (K) hat folgende durchschnittliche Zusammensetzung:

Mengenstrom (K) ( 21,399 kg/h )
0,8 % Polyarylamin
26,6 % Anilin
6,6 % Chlorwasserstoff
66,0 % Wasser.

Mengenstrom (K) wird geteilt in einen ersten Teilstrom, welcher in einem Wasserverdampfer (8) durch Abdestillieren von Wasser (Mengenstrom X ; ca. 2,9 kg/h) aufkonzentriert wird, und danach als Mengenstrom (U) der Extraktionsstufe (4) zugeführt wird,

Mengenstrom (U) ( 11,375 kg/h )
1,0 % Polyarylamin
33,3 % Anilin
8,2 % Chlorwasserstoff
57,5 % Wasser.

und einem zweiten Teilstrom, welcher direkt, d.h. mit der Zusammensetzung von Mengenstrom (K) der Extraktionsstufe (3) zugeführt wird.

In dem mehrstufig wirkenden Extraktor (3) wird der wässrige Teilstrom von (K) bei 90°C einer organischen Phase (O), im vorliegenden Fall identisch nach Menge und Zusammensetzung mit Mengenstrom (D), entgegengeführt.

Die im Extraktor (3) resultierende wässrige Phase (Q) wird mit der wässrigen Phase (U) vereinigt unter Bildung der wässrigen Phase (C).

Mengenstrom (Q) ( 7,932 kg/h )
24,1 % Polyarylamin
10,0 % Anilin
5,9 % Chlorwasserstoff
60,0 % Wasser.

Die im Extraktor (3) resultierende organische Phase (P) hat folgende durchschnittliche Zusammensetzung:

Mengenstrom (P) ( 11,374 kg/h )
7,9 % Polyarylamin
33,6 % Anilin
57,4 % Xylol
< 0,1 % Chlorwasserstoff
ca. 1,0 % Wasser.

In einer als mehrstufiger Extraktor ausgeführten Waschstufe (6.0) wird der Mengenstrom (P) im Gegenstrom mit Wasser aus Mengenstrom (X) (ca. 0,9 kg/h) gewaschen.

Zur Sicherheit wird der gewaschene Mengenstrom (P) mit verdünnter Natronlauge gewaschen. Die wässrige Phase wird als Abwasser entsorgt.

Der gewaschene und von Säureresten befreite Mengenstrom (P) wird in einer ersten Destillationsstufe (6.1) vom größten Teil des Xylols und einem Teil des Anilins befreit. Das Destillat (Mengenstrom E) wird zur Bildung von Mengenstrom (B) verwendet.

In einer zweiten Destillationsstufe (6.2) wird aus der Sumpfphase von (6.1) das Anilin und restliches Xylol abdestilliert. Das Destillat wird aufgeteilt auf die Mengenströme (B) und (J).

Als Destillationssumpf von Stufe (6.2) verbleibt ein Polyamingemisch, welches als Mengenstrom (G) mit ca. 0,90 kg/h in Tank (9) gesammelt wird.

Die über die Sicherheitsneutralisationsstufen bei der Wäsche mit Natronlauge dem System entzogenen Säuremengen werden durch Zugabe zum Mengenstrom (C) von außen ersetzt, entzogene Wassermengen durch Zugabe zum Mengenstrom (X).

| Polyarylamin<br><br>GC: | A<br>[Gew. - %] | G<br>[Gew. - %] | N<br>[Gew. - %] |
|---|---|---|---|
| 2,2' Diamino-diphenylmethan | 0,22 | 1,20 | ---- |
| 2,4'-Diamino-diphenylmethan | 7,12 | 35,20 | 0,80 |
| 4,4'-Diamino-diphenylmethan | 60,20 | 26,40 | 67,80 |
| N-Methyl-4,4'-diamino-diphenylmethan | 0,21 | 0,70 | 0,10 |
| Σ Diamino-diphenylmethane | 67,74 | 63,50 | 68,70 |
| Σ Mehrkernpolyamine | 32,26 | 36,50 | 31,30 |
| Mengenverteilung | 100 % | 18,4 % | 81,6 % |

## Patentansprüche

1. Verfahren zur Fraktionierung und Reiningung von aromatischen Polyamingemischen) der Diphenylmethanreihe, dadurch gekennzeichnet, daß man

   a) das Polyaminausgangsgemisch (A) in einem zweiphasigen System, bestehend aus (i) einer hydrophoben Lösungsmittelphase (B), die im wesentlichen aus hydrophobem Lösungsmittel, und (ii) einer wäßrigen Phase (C), bestehend im wesentlichen aus Wasser, einer starken Säure und zumindest teilweise in der Salzform vorliegendem Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, unter Zuhilfenahme einer nach dem Gegenstromprinzip arbeitenden Extraktionsstufe (4) unter Durchmischung der Phasen verteilt, indem man das Ausgangspolyamingemisch über die wäßrige Phase in die Extraktionsstufe (4) einbringt und die die Extraktionsstufe (4) verlassende organische Phase (D)

   b) zumindest teilweise über eine zwischengeschaltete Extraktionsstufe (3) und/oder

   c) unter Abtrennung eines Teilstromes vor oder nach der durchlaufenen Extraktionsstufe (3) und Rückführung des abgetrennten Teilstromes zur Extraktionsstufe (4) über eine vorgeschaltete Extraktionsstufe (2)

d) in einer mehrstufigen Destillation (6.1), (6.2) in eine erste Fraktion (E), bestehend im wesentlichen aus hydrophobem Lösungsmittel, eine zweite Fraktion (F), bestehend im wesentlichen aus Hilfsamin und einem Destillationsrückstand (G), bestehend im wesentlichen aus einer ersten Polyaminfraktion, auftrennt und

e) die die Extraktionsstufe (4) verlassende wäßrige Phase (H) in eine Extraktionsstufe (5) leitet, in welcher nach dem Prinzip der Gegenstromextraktion eine Extraktion der wäßrigen Phase mit einer aus Lösungsmittel und Hilfsamin bestehenden Lösungsmittelphase (J) erfolgt, und die an Polyarylamin verarmte wäßrige Phase (K) resultiert, die

f) zumindest teilweise über eine zwischengeschaltete Destillationsstufe (8)

g) zumindest teilweise über eine zwischengeschaltete Extraktionsstufe (3) und/oder

h) zumindest teilweise zunächst über eine vorgeschaltete Extraktionsstufe (2) und anschließend

i) zur Extraktionsstufe (4) zurückgeführt und dort als Mengenstrom (C) wiederverwendet wird und man

j) die in der Extraktionsstufe (5) anfallende organische Phase (L) in ein aus hydrophobem Lösungsmittel und Hilfsamin bestehendes Destillat (M) und einen, aus einer zweiten Polyaminfraktion bestehenden Destillationsrückstand (N) zerlegt, wonach das Destillat (M) mit zumindest einer Teilmenge des in der zweiten Destillationsstufe (6.2) der organischen Phase (D) gewonnenen Destillates (F) vereinigt und anschließend zur Extraktionsstufe (5) zurückgeführt und dort wiederverwendet wird.

**2.** Verfahren gemäß Anspruch 1, dadurch gekennzeichnet, daß man

f) die die Extraktionsstufe (5) verlassende wäßrige Phase (K) vor ihrer Wiederverwendung zumindest teilweise destillativ (8) von einem Teil (X) des in ihr enthaltenen Wassers befreit, dieses zum Waschen (6.0) des der destillativen Aufarbeitung (6.1), (6.2) zugeführten Teiles der die Extraktionsstufe (4) verlassenden organischen Phase (D) und/oder der die Extraktionsstufe (3) verlassenden organischen Phase (P) und/oder zum Waschen (7.0) des der destillativen Aufarbeitung (7.1) zugeführten Teiles der die Extraktionsstufe (5) verlassenden organischen Phase (L) zwecks Entfernens von Säurespuren verwendet, das hierbei anfallende Wasser (Y) an geeigneter Stelle in die wäßrige Phase zurückführt, die resultierende konzentrierte wäßrige Phase mit dem verbliebenen Rest von (K) vereiningt und als Mengenstrom (C) der Wiederverwendung zuführt.

**3.** Verfahren gemäß Anspruch 1 oder 2, dadurch gekennzeichnet, daß man

b) die in der Extraktionsstufe (4) anfallende organische Phase (D) zumindest teilweise in einer zwischengeschalteten Extraktionsstufe (3) mit zumindest einer Teilmenge des Mengenstrom (C) im Gegenstrom extrahiert und/oder
mit zumindest einer Teilmenge der in der vorhandenen vorgeschalteten Extraktionsstufe (2) anfallenden wäßrigen Phase im Gegenstrom extrahiert,
die in der zwischengeschalteten Extraktionsstufe (3) resultierende wäßrige Phase der Extraktionsstufe (4) zuführt und die in der zwischengeschalteten Extraktionsstufe (3) anfallende organische Phase der Aufarbeitungsstufe (6) zuführt.

**4.** Verfahren gemäß einem oder mehreren der Ansprüche 1 bis 3, dadurch gekennzeichnet, daß man

c) einen Teilstrom der die Extraktionsstufe (4) verlassenden organischen Phase (D) und/oder einen Teilstrom der die vorhandene zwischengeschaltete Extraktionsstufe (3) verlassenden organischen Phase abtrennt,
und in einer vorgeschalteten Extraktionsstufe (2) mit einer Teilmenge, vorzugsweise mit der Gesamtmenge der als Mengenstrom (C) zur Verfügung stehenden wäßrigen Phase im Gegenstrom extrahiert,
den in der Extraktionsstufe (2) eingesetzten organischen Mengenstrom so bemißt, daß in (2) ein möglichst weitgehender Übergang des im besagtem organischen Mengenstrom enthaltenen Polyamins in die wäßrige Phase erfolgt,
die in der vorgeschalteten Extraktionsstufe (2) resultierende wäßrige Phase nach Zugabe von Wasser aus Mengenstrom (Y) und/oder Hilfsamin der Extraktionsstufe (3) zuführt und
die in der vorgeschalteten Extraktionsstufe (2) anfallende an Polyamin verarmte organische Phase (T) der Extraktionsstufe (4) zuführt.

5. Verfahren gemäß einem oder mehreren der Ansprüche 1 bis 4, dadurch gekennzeichnet, daß man als Hilfsamin Anilin verwendet.

6. Verfahren gemäß einem oder mehreren der Ansprüche 1 bis 5, dadurch gekennzeichnet, daß man ein Polyamingemisch der Diphenylmethanreihe als Polyamingemisch verwendet, wie es bei der säurekatalysierten Anilin\-Formaldehydkondensation anfällt.

7. Verfahren zur Herstellung der entsprechenden aromatischen Polyisocyanatgemische, dadurch gkennzeichnet, daß gemäß den Ansprüchen 1 bis 6 behandelte aromatische Polyamingemische verwendet werden.

8. Verfahren zur Herstellung kernhydrierte Polyamine oder Vernetzer und Epoxidhärter, dadurch gekennzeichnet, daß gemäß den Ansprüchen 1 bis 6 behandelte aromatische Polyamingemische verwendet werden.

9. Verfahren zur Herstellung von Polyurethankunststoffen, dadurch gekennzeichnet, daß gemäß den Ansprüchen 1 bis 6 behandelte aromatische Polyamingemische verwendet werden.

**Claims**

1. Process for the fractionation and purification of aromatic polyamine mixtures of the diphenylmethane series, characterised in that

a) the polyamine starting mixture (A) is distributed in a two-phase system consisting of (i) a hydrophobic solvent phase (B) substantially consisting of hydrophobic solvent and (ii) an aqueous phase (C) which consists substantially of water, a strong acid and auxiliary amine present at least partially in the salt form, which amine is virtually insoluble in water and under standard pressure has a boiling point at least 20°C below the boiling point of the lowest boiling component of the starting mixture and at least 20°C above the boiling point of the solvent, by means of an extraction step (4) operating according to the counter-current principle with thorough mixing of the phases, by introducing the starting polyamine mixture into the extraction step (4) via the aqueous phase, and the organic phase (D) leaving the extraction step (4) is separated

b) at least partially via an intermediate extraction step (3) and/or

c) with separation off of a sub-stream before or after the continous extraction step (3) which has been passed through, and return of the separated sub-stream to the extraction step (4) via an upstream extraction step (2)

d) in a multi-step distillation (6.1), (6.2) into a first fraction (E) which consists substantially of hydrophobic solvent, a second fraction (F) consisting substantially of auxiliary amine and a distillation residue (G) consisting substantially of a first polyamine fraction, and

e) the aqueous phase (H) leaving the extraction step (4) is fed to an extraction step (5) in which an extraction of the aqueous phase with a solvent phase (J) consisting of solvent and auxiliary amine is carried out according to the principle of counter-current extraction, and the aqueous phase (K) depleted in polyarylamine results, which is returned

f) at least partially via an intermediate distillation step (8)

g) at least partially via an intermediate extraction step (3) and/or

h) at least partially, initially via an upstream extraction step (2) and subsequently

i) to the extraction step (4) where it is used again as stream (C) and

j) the organic phase (L) obtained in the extraction step (5) is separated into a distillate (M) consisting of hydrophobic solvent and auxiliary amine and a distillation residue (N) consisting of a second polyamine fraction, whereupon the distillate (M) is combined with at least a sub-quantity of the distillate (F) obtained in the second distillation step (6.2) of the organic phase (D) and is subsequently returned to the extraction step (5) where it is used again.

2. Process according to claim 1, characterised in that

f) before being reused, the aqueous phase (K) leaving the extraction step (5) is freed at least partially from a part (X) of the water contained therein by distillation (8), said water being used for washing (6.0) that part of the organic phase (D) leaving the extraction step (4) and/or of the organic phase (P) leaving the extraction step (3) fed to work-up by distillation (6.1), (6.2), and/or for washing (7.0) that part of the organic phase (L) leaving the extraction step (5) fed to work-up by distillation (7.1) for the purpose of removing acid traces, the water (Y) obtained in so doing is returned to the aqueous phase at a suitable place and the resulting concentrated aqueous phase is combined with the remaining residue of (K) and reused as stream (C).

3. Process according to claim 1 or 2, characterised in that

b) the organic phase (D) obtained in the extraction step (4) is extracted in counter-current at least partially in an intermediate extraction step (3) with at least a sub-quantity of stream (C) and/or is extracted in counter-current with at least a sub-quantity of the aqueous phase obtained in the upstream extraction step (2), the aqueous phase resulting in the intermediate extraction step (3) is fed to the extraction step (4) and the organic phase obtained in the intermediate extraction step (3) is fed to the work-up step (6).

4. Process according to one or more of claims 1 to 3, characterised in that

c) a sub-stream of the organic phase (D) leaving the extraction step (4) and/or a sub-stream of the organic phase leaving the intermediate extraction step (3) is separated,
and extracted in counter-current in an upstream extraction step (2) with a sub-quantity, preferably with the entire quantity, of the aqueous phase available as stream (C),
the organic stream used in the extraction step (2) is metered such that the most extensive transfer possible of the polyamine contained in the above-mentioned organic stream to the aqueous phase takes place in (2),
the aqueous phase resulting in the upstream extraction step (2) is fed to the extraction step (3) after the addition of water from stream (Y) and/or auxiliary amine, and
the organic phase (T) obtained in the upstream extraction step (2) and depleted in polyamine is fed to the extraction step (4).

5. Process according to one or more of claims 1 to 4, characterised in that the auxiliary amine used is aniline.

6. Process according to one or more of claims 1 to 5, characterised in that the polyamine mixture used is a polyamine mixture of the diphenylmethane series as is obtained from acid-catalysed aniline/formaldehyde condensation.

7. Process for the production of the corresponding polyisocyanate mixtures, characterised in that aromatic polyamine mixtures treated according to claims 1 to 6 are used.

8. Process for the production of ring-hydrogenated polyamines or crosslinking agents and epoxy hardeners, characterised in that aromatic polyamine mixtures treated according to claims 1 to 6 are used.

9. Process for the production of polyurethane plastics, characterised in that aromatic polyamine mixtures treated according to claims 1 to 6 are used.

**Revendications**

1. Procédé pour fractionner et purifier des mélanges de polyamines aromatiques de la série du diphénylméthane, caractérisé en ce que

a) on partage le mélange des polyamines de départ (A) dans un système à deux phases consistant en (i) une phase de solvant hydrophobe (B) qui consiste essentiellement en un solvant hydrophobe et (ii) une phase aqueuse (C) consistant essentiellement en eau, un acide fort et une amine auxiliaire présente en partie au moins à l'état de sel, cette amine étant pratiquement insoluble dans l'eau et ayant, à pression normale, un point d'ébullition situé à au moins 20°C au-dessous du point d'ébullition du composant bouillant le plus bas du mélange initial et à au moins 20°C au-dessus du point d'ébullition du solvant, grâce à un stade d'extraction (4) opérant selon le principe du contre-courant, avec mélange des phases, en introduisant le mélange des polyamines de départ, par l'intermédiaire de la phase aqueuse, au stade d'extraction (4), et on sépare la phase

organique (D) quittant le stade d'extraction (4)

b) en passant en partie au moins par l'intermédiaire d'un stade d'extraction (3) et/ou

c) en séparant un courant partiel avant ou après passage au stade d'extraction (3) et en recyclant le courant partiel séparé au stade d'extraction (4) par l'intermédiaire d'un stade d'extraction préalable (2),

d) dans une distillation à plusieurs étages (6.1), (6.2), en une première fraction (E) consistant essentiellement en solvant hydrophobe, une deuxième fraction (F) consistant essentiellement en amine auxiliaire et un résidu de distillation (G) consistant essentiellement en une première fraction de polyamines, et

e) on envoie la phase aqueuse (H) quittant le stade d'extraction (4) dans un stade d'extraction (5), dans lequel, selon le principe de l'extraction à contre-courant, il y a extraction de' la phase aqueuse par une phase solvant (I) consistant en solvant et amine auxiliaire, ce qui donne une phase aqueuse (K) appauvrie en polyarylamines qu'on recycle,

f) en partie au moins en passant par un étage de distillation intermédiaire (8)

g) en partie au moins en passant par un stade d'extraction intermédiaire (3) et/ou

h) en partie au moins en passant d'abord par un stade d'extraction préalable (2) puis

i) au stade d'extraction (4) où elle est réutilisée en tant que courant (C) et

j) on fractionne la phase organique (L) sortant du stade d'extraction (5) en un distillat consistant en solvant hydrophobe et amine auxiliaire (M) et un résidu de distillation consistant en une deuxième fraction de polyamines (N), après quoi on combine le distillat (M) avec une partie au moins du distillat (F) obtenu au deuxième étage de distillation (6.2) de la phase organique (D) et on recycle au stade d'extraction (5) où on le réutilise.

**2.** Procédé selon revendication 1, caractérisé en ce que

f) la phase aqueuse (K) sortant du stade d'extraction (5), avant réutilisation, est débarrassée, en partie au moins, par distillation (8) d'une partie (X) de l'eau qu'elle contient, laquelle sert au lavage (6.0) de la partie de la phase organique (D) quittant le stade d'extraction (4) et envoyée à la distillation (6.1), (6.2) et/ou de la phase organique (P) quittant le stade d'extraction (3) et/ou pour le lavage (7.0) de la partie de la phase organique (L) quittant le stade d'extraction (5) et envoyée à la distillation (7.1) en vue de l'élimination des traces d'acide, l'eau (Y) sortante est recyclée à un endroit approprié dans la phase aqueuse, la phase aqueuse concentrée résultante est combinée avec le reste de (K) et envoyée en tant que courant (C) à la réutilisation.

**3.** Procédé selon revendication 1 ou 2, caractérisé en ce que

b) la phase organique (D) quittant le stade d'extraction (4) est extraite en partie au moins dans un stade d'extraction intermédiaire (3) par une partie au moins du courant (C), à contre-courant, et/ou
par une partie au moins de la phase aqueuse sortant du stade d'extraction préalable (2), à contre-courant,
la phase aqueuse sortant du stade d'extraction intermédiaire (3) est envoyée au stade d'extraction (4) et la phase organique sortant du stade d'extraction intermédiaire (3) est envoyée au stade isolement (6).

**4.** Procédé selon une ou plusieurs des revendications 1 à 3, caractérisé en ce que

c) on sépare un courant partiel de la phase organique (D) quittant le stade d'extraction (4) et/ou un courant partiel de la phase organique quittant le stade d'extraction intermédiaire (3),
et on l'extrait dans un stade d'extraction préalable (2) par une partie mais de préférence par la totalité de la phase aqueuse dont on dispose sous la forme du courant (C), à contre-courant,
on règle le débit du courant organique envoyé au stade d'extraction (2) en sorte que, à ce stade, il se produise un transfert aussi complet que possible des polyamines contenues dans ledit courant organique vers la phase aqueuse,
on envoie la phase aqueuse sortant du stade d'extraction préalable (2), après addition d'eau du courant (Y) et/ou d'amine auxiliaire, au stade d'extraction (3) et
on envoie la phase organique (T) appauvrie en polyamines quittant le stade d'extraction préalable (2) au stade d'extraction (4).

**5.** Procédé selon une ou plusieurs des revendications 1 à 4, caractérisé en ce que l'amine auxiliaire utilisée est l'aniline.

**6.** Procédé selon une ou plusieurs des revendications 1 à 5, caractérisé en ce que le mélange de polyamines mis en oeuvre est un mélange de polyamines de la série du diphénylméthane tel qu'obtenu par condensation aniline-formaldéhyde catalysée par un acide.

7. Procédé de préparation de mélanges de polyisocyanates aromatiques, caractérisé en ce que l'on utilise des mélanges de polyamines aromatiques traités selon revendications 1 à 6.

8. Procédé de préparation de polyamines hydrogénées dans les noyaux ou d'agents réticulants et durcisseurs d'époxydes, caractérisé en ce que l'on utilise des mélanges de polyamines aromatiques traités selon les revendications 1 à 6.

9. Procédé de préparation de résines synthétiques de polyuréthanes, caractérisé en ce que l'on utilise des mélanges de polyamines aromatiques traités selon les revendications 1 à 6.

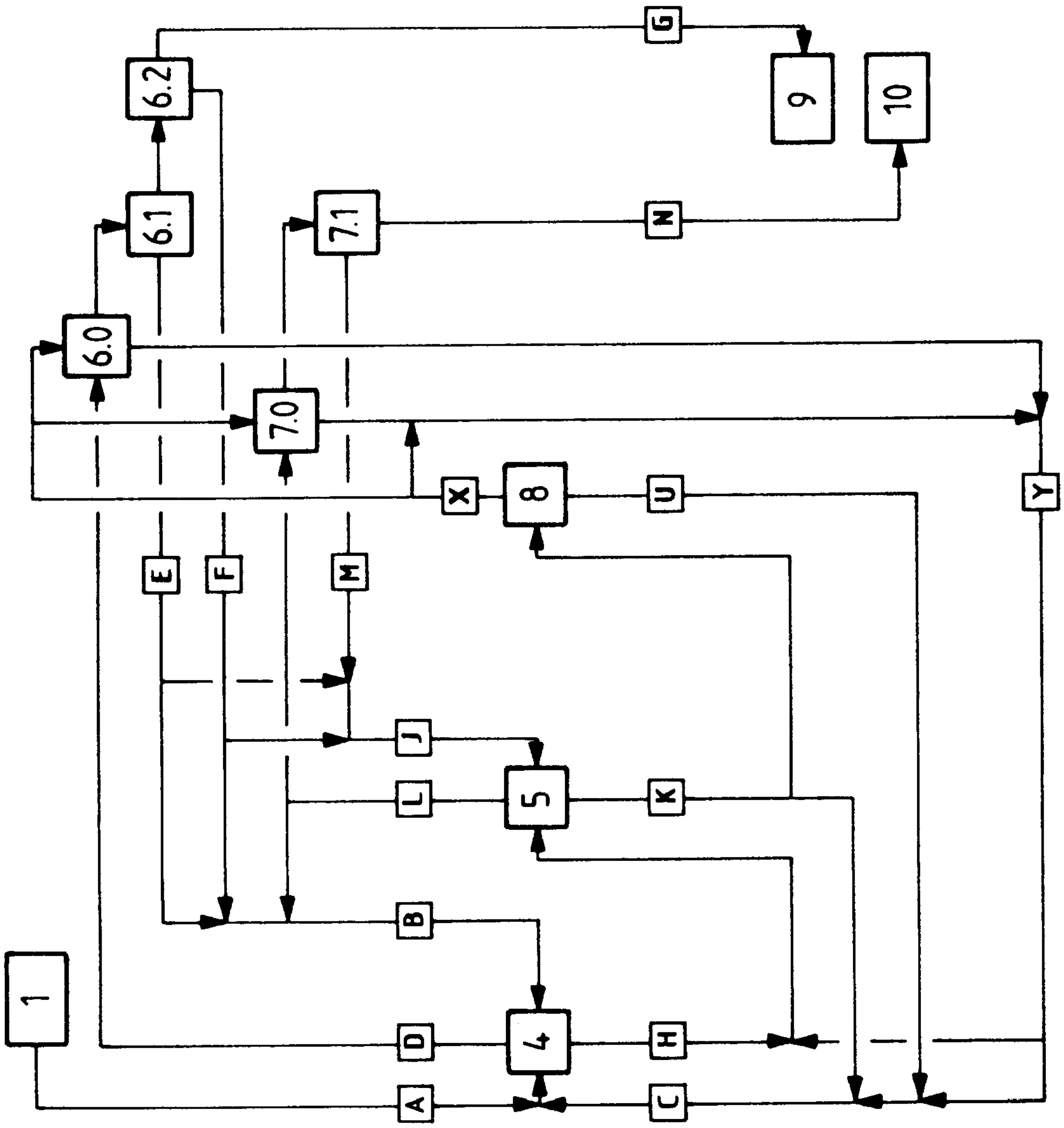


Fig.1

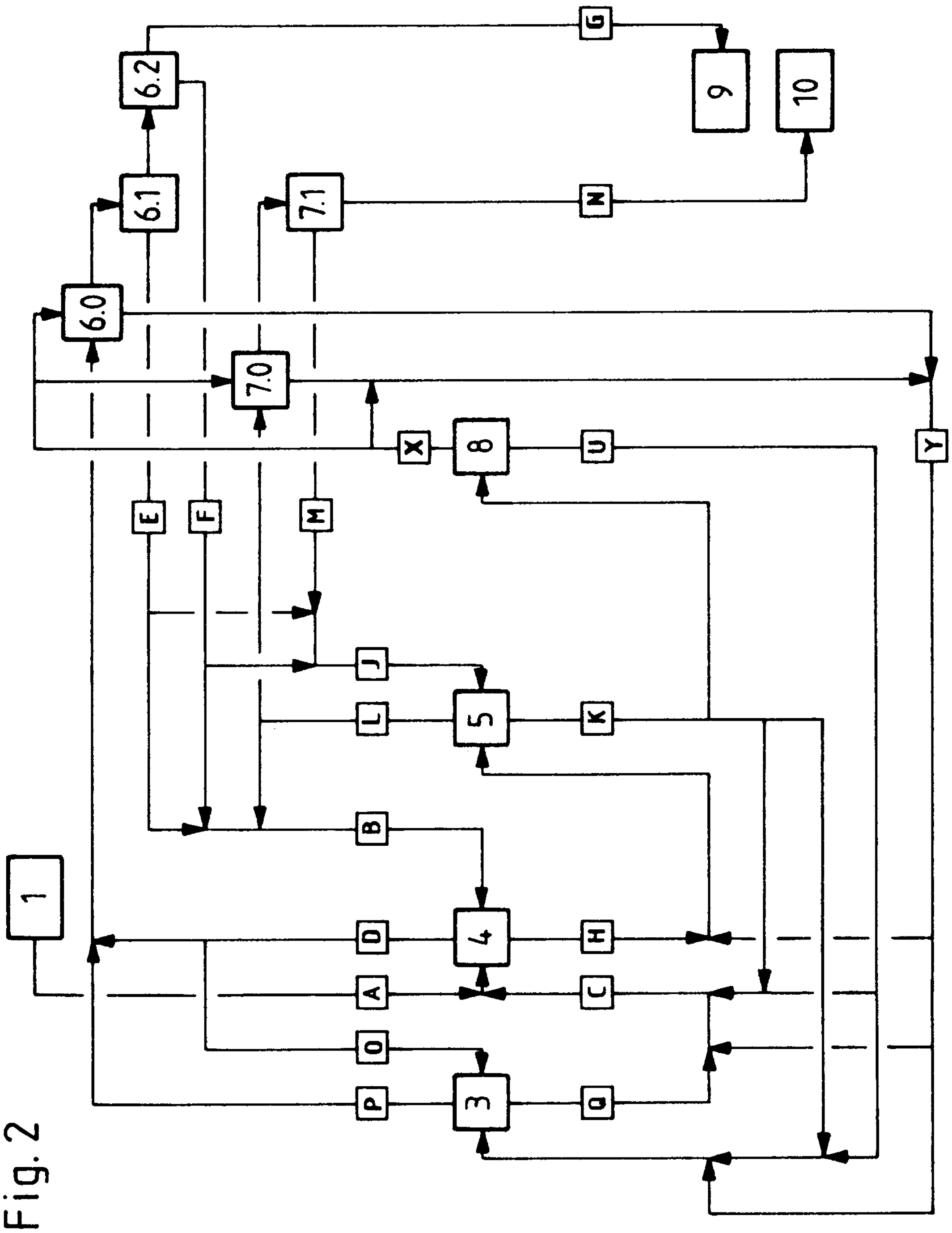


Fig. 2

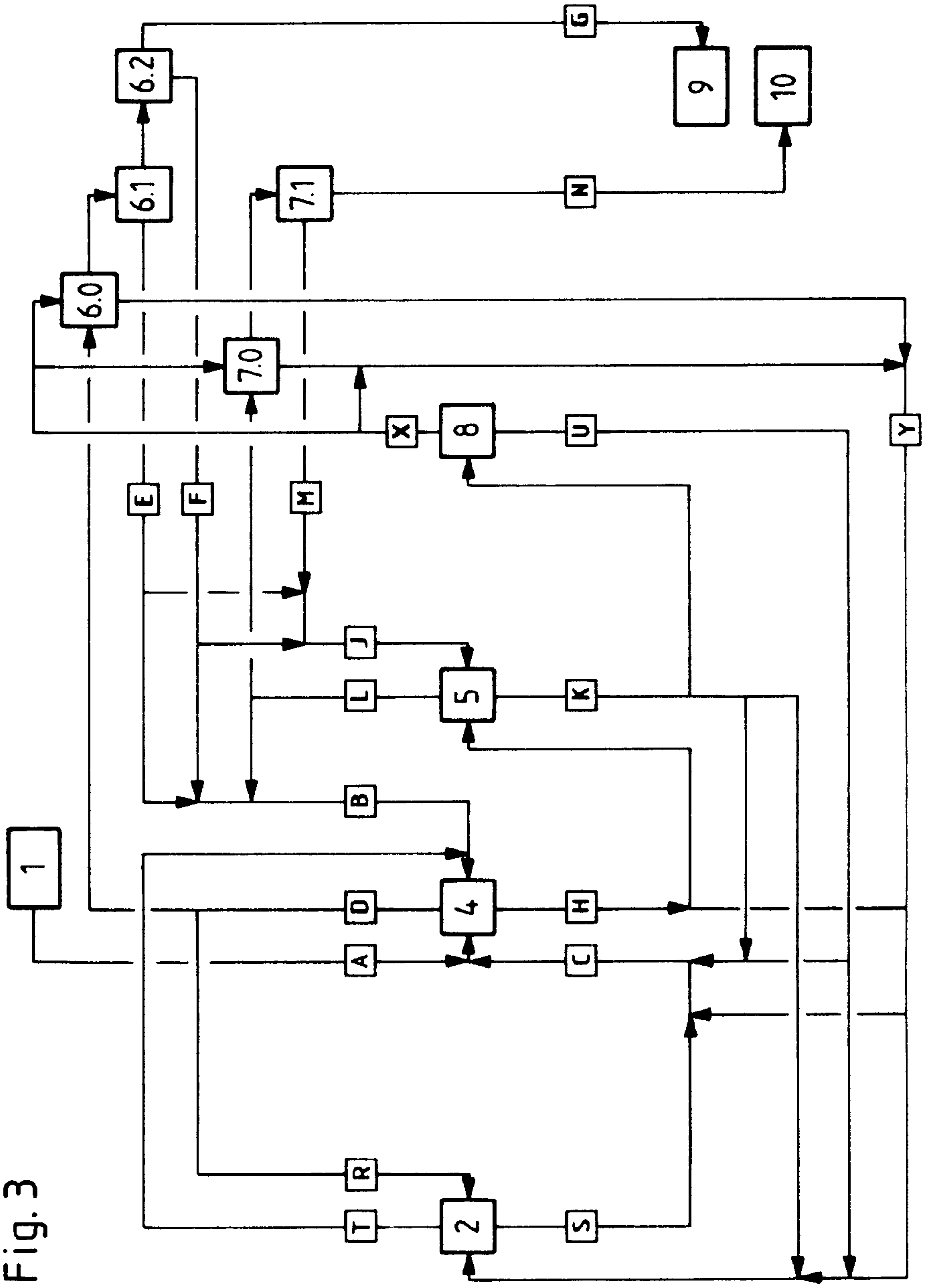


Fig. 3

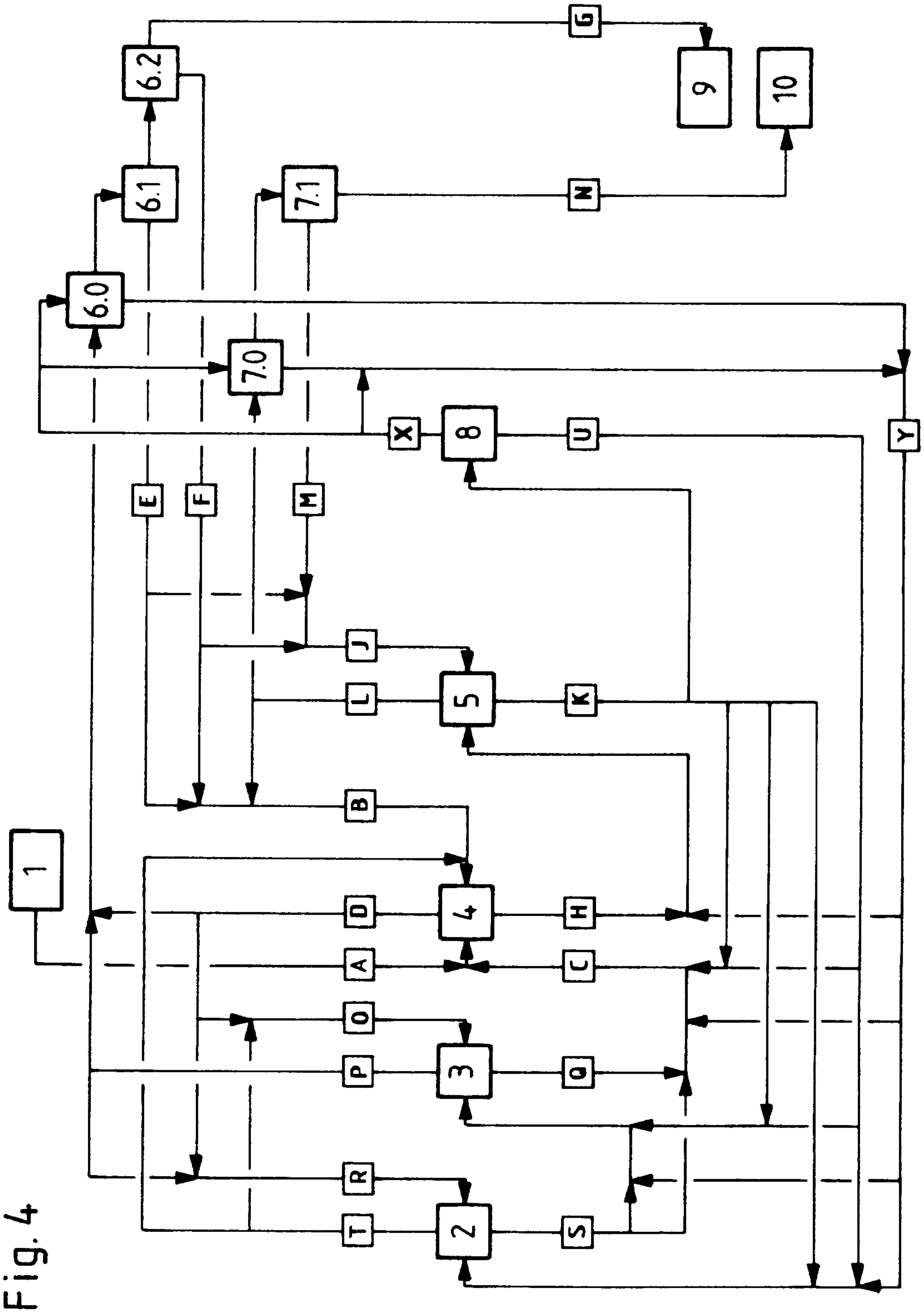


Fig. 4